# EUROPEAN PATENT SPECIFICATION

(11) **EP 4 578 891 B1**
(45) Date of publication and mention of the grant of the patent: **22.04.2026**
(21) Application number: 23856315.9
(22) Date of filing: 30.06.2023
(51) Int. Cl.: C08G 65/323, C08G 65/331, C08G 65/337

(54) **METHOD FOR PREPARING POLYETHYLENE GLYCOL ALDEHYDE DERIVATIVES**
VERFAHREN ZUR HERSTELLUNG VON POLYETHYLENGLYKOLALDEHYDDERIVATEN
PROCÉDÉ DE PRÉPARATION DE DÉRIVÉS D'ALDÉHYDE DE POLYÉTHYLÈNE GLYCOL

(30) Priority: 26.08.2022 CN 202211032645
(43) Date of publication of application: 02.07.2025
(73) Proprietor: Xiamen Sinopeg Biotech Co., Ltd., Xiamen, Fujian 361100 (CN)
(72) Inventor: LIN, Sheng, Xiamen, Fujian 361100 (CN); WANG, Ailan, Xiamen, Fujian 361100 (CN); ZHU, Qi, Xiamen, Fujian 361100 (CN); WENG, Wengui, Xiamen, Fujian 361100 (CN); LIU, Chao, Xiamen, Fujian 361100 (CN)
(74) Representative: Algemeen Octrooi- en Merkenbureau B.V.
(86) International application number: PCT/CN2023/105409
(87) International publication number: WO 2024/041225

(56) References cited:
- EP-A1- 1 591 467
- WO-A1-2014/110867
- WO-A1-2017/146443
- CA-A1- 2 724 823
- CN-A- 101 376 708
- CN-A- 104 927 044
- CN-A- 108 530 637
- CN-A- 114 409 890
- CN-A- 114 479 059
- CN-A- 114 507 342
- CN-A- 115 417 984
- US-A1- 2022 118 100

## Description

### TECHNICAL FIELD

The present application relates to the field of macromolecular synthetic chemistry, especially to a method for preparing a polyethylene glycol acetal derivative or a polyethylene glycol aldehyde derivative.

### BACKGROUND

PEGylation is one of the most important means of modification of drugs or bio-related substances, and the modified drug molecules will acquire many excellent properties of polyethylene glycol (e.g., hydrophilicity, flexibility, anticoagulant properties, etc.). Wherein, the polyethylene glycol aldehyde modifiers with an aldehyde as the terminal group are highly important modifiers in the field of proteins, which have the advantages of high selectivity and high activity retention. The aldehyde group has a certain selectivity for the N-terminus of protein, because the PKa of the N-terminal amino group of a protein is lower than that of the side chain amine group. When the side chain amino group is protonated at a certain pH and loses the ability to perform nucleophilic attack on aldehyde groups, the N-terminal amino group remains unprotonated and retains its nucleophilic attack ability. Aldehyde groups can also form a Schiff base with the N-terminal amino group of a protein, which, after reduction, results in a stable imine linkage while preserving the positive charge of the amino group, playing an important role in maintaining the structure and activity of proteins.

Prior art US6465694B1 discloses that polyethylene glycol aldehyde can be obtained by oxidizing the terminal hydroxyl group of polyethylene glycol, e.g., by adding oxygen to a mixture of PEG and a catalyst to oxidize the -CH₂OH group to -CHO. However, under most oxidizing conditions, the PEG chain tends to decompose, leading to inefficient conversion of the terminus of the PEG chain. Polyethylene glycol aldehyde can also be prepared using acetals, obtains by introducing a linear acetal group at the end of the PEG chain and subsequent hydrolyzing, but the linear acetal used as a starting material in this method is unstable and may produce by-products. Specifically, CN1763122A discloses that the alkali-catalyzed reaction between 3-hydroxypropanal diethyl acetal and PEG methanesulfonate will produce a large amount of unstable PEG vinyl ether in the side reaction, with a reaction yield of less than 85%-90%; additionally, intermolecular coupling may occur, leading to low purity of the resulting polyethylene glycol aldehyde product, thus affecting the modification efficiency of polyethylene glycol aldehyde on proteins and other drugs. CN102037056A discloses a method of preparing a high-purity polyethylene glycol aldehyde, but it is not suitable for commercialization because of the cumbersome reaction steps.

CN114507342A discloses a Williamson condensation reaction method for synthesizing mPEG glyceraldehyde acetal derivative and this glyceraldehyde acetal group is subsequently hydrolyzed to a diol with high conversion rate through synthesis. EP1591467A1 discloses water-soluble polymer alkanals, specifically polymers comprising an aldehyde functionality coupled to a polymer segment by one or more carbon atoms, such as polymer butanals. The document also describes methods for preparing these polymer alkanals and their subsequent use in forming conjugates with biologically active agents, for example, human growth hormone (hGH). US2022/118100A1 relates to a monofunctional branched polyethylene glycol (PEG) and a bio-related substance modified by it. The branched PEG described has a general formula comprising two branch chains and a main chain, with an active functional group located at the terminal end of the main chain. CN101376708A discloses a two-step method for synthesizing a polyalkyl ether polymer having a propionaldehyde group as a terminal group. The method comprises reacting a polyalkyl ether polymer with a leaving group-substituted dimethoxy (or ethoxy) propional to form a polyacetal-substituted polymer, which is subsequently hydrolyzed in an acidic aqueous solution to yield the final propionaldehyde-substituted polymer.

Therefore, it is necessary to improve the existing preparation methods and develop a method with fewer reaction steps to obtain the polyethylene glycol aldehyde derivatives with high terminal substitution rate, high yields, and high purity.

### SUMMARY

To achieve the above objectives, the present application provides a method for preparing the polyethylene glycol acetal derivative represented by formula (1) or formula (2), and the embodiment is as follows:
A method for preparing a polyethylene glycol acetal derivative, wherein the structure of the derivative is represented by formula (1) or formula (2): 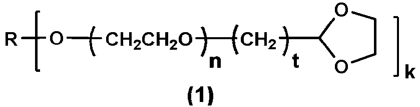 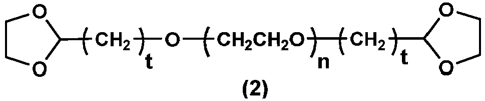
wherein, n is an integer from 20 to 1000;
t is independently an integer from 1 to 6 at each occurrence;
k is an integer from 1 to 8; when k is 1, R is selected from the group consisting of H, -CH₃, -CH₂CH₃, -TBS, -Bn, -(CH₂)ₜₚ-COOH, -(CH₂)ₜₚ-COOtBu and -(CH₂)ₜₚ-N₃; tp is an integer from 1 to 3; when k is an integer from 2 to 8, R is a multivalent branched structure, ranging from divalent to octavalent;
the preparation method comprises:
   dissolving small molecule acetal derivative **I-2** in an organic solvent; activating **I-2** with an alkali reagent; adding polyethylene glycol derivative **I-1** or **I-3**, each of which contains functional group X₁ and dissolving in an organic solvent; then conducting the reaction for 2 to 24 hours at 20 to 90°C to obtain the polyethylene glycol acetal derivative represented by formula (1) or formula (2); the alkali reagent is sodium hydroxide or potassium hydroxide; wherein, X₁ is independently a *p*-toluenesulfonyl group or a methanesulfonyl group at each occurrence; X₂ is -OH;

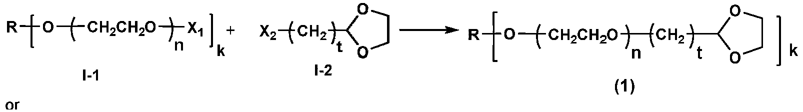

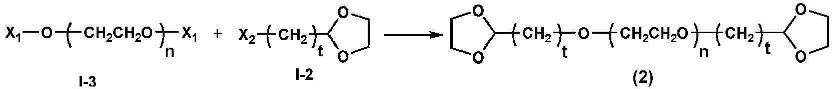

The present application also provides a method for preparing a polyethylene glycol aldehyde derivative by subjecting the acetal derivative represented by formula (1) or (2) to acid treatment, wherein the acetal derivative represented by formula (1) or (2) is prepared according to the aforementioned method.

The present application also provides a bio-related substance modified with a polyethylene glycol aldehyde derivative obtained according to the aforementioned preparation method; wherein, the bio-related substance is selected from the group consisting of a peptide, a polypeptide, a protein, a polysaccharide, a steroid, a nucleotide, an oligonucleotide, a polynucleotide, and a lipid.

### THE BENEFICIAL EFFECTS OF THE PRESENT APPLICATION

The present application provides a method for preparing polyethylene glycol acetals and derivatives thereof, wherein a series of linear and nonlinear polyethylene glycol aldehyde derivatives with single or multiple aldehyde functionalization are prepared using small molecule cyclic acetal derivatives and polyethylene glycol as starting materials in the presence of an alkali reagent. The preparation method of the present application features fewer reaction steps and eliminates the need for column chromatography for separation and purification, with no detectable amount of residual polyethylene glycol starting materials or other by-products. The method has advantages such as stability, high efficiency, environmental friendliness, cost-effectiveness, and suitability for large-scale production. The resulting products exhibit high purity and a high substitution rate of terminal functional groups, providing superior polyethylene glycol aldehyde modifiers in the field of PEGylation modification.

### BRIEF DESCRIPTION OF THE DRAWINGS

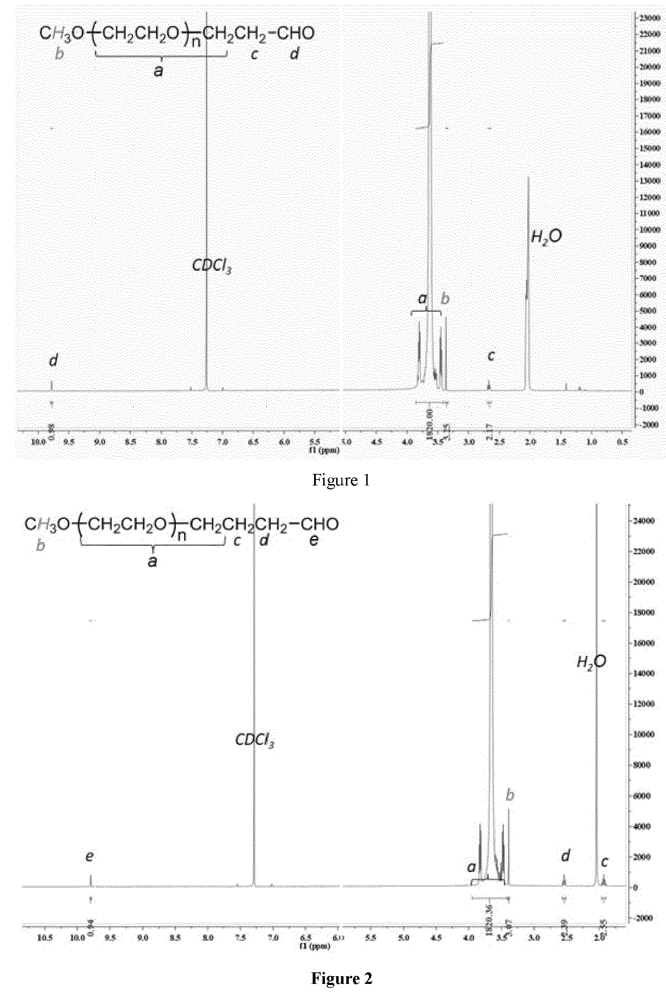

Figure 1 is the NMR spectrum (¹HNMR) of mPEG-propionaldehyde-20k (P1-2) prepared in Example-1 of the present application.
Figure 2 is the NMR spectrum (¹HNMR) of mPEG-butyraldehyde-20k (P2-2) prepared in Example-2 of the present application.

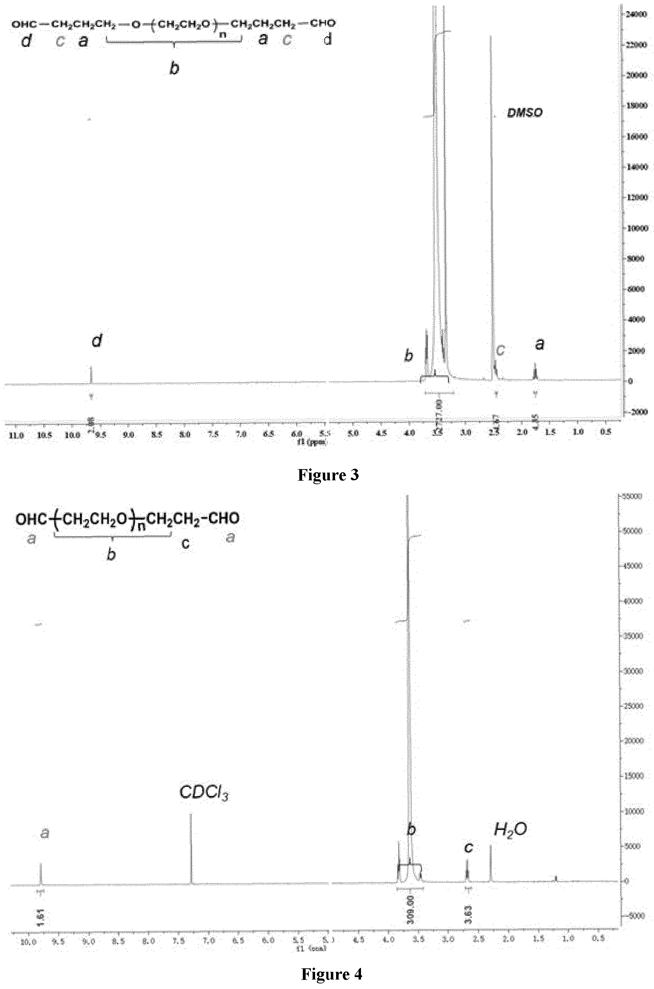

Figure 3 is the NMR spectrum (¹HNMR) of PEG-dibutyraldehyde-30k (P3-2) prepared in Example-3 of the present application.
Figure 4 is the NMR spectrum (¹HNMR) of PEG-dipropionaldehyde-3.4k (P4-2) prepared in Example-4 of the present application.

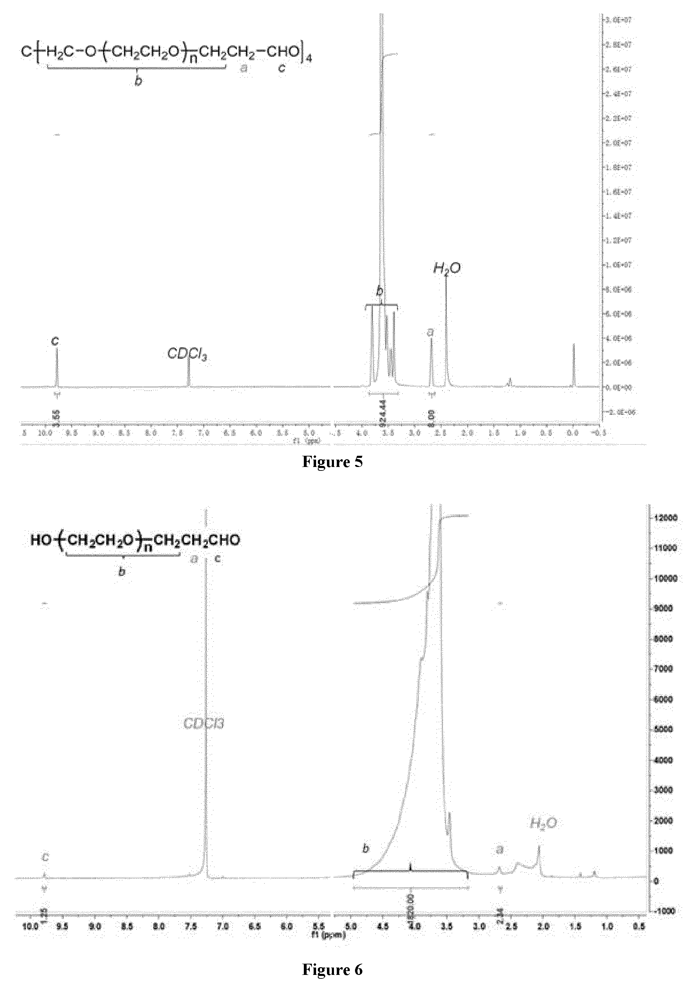

Figure 5 is the NMR spectrum (¹HNMR) of 4-arm PEG-propionaldehyde-10k (P5-2) prepared in Example-5 of the present application.
Figure 6 is the NMR spectrum of HO-PEG-propionaldehyde-20k (P6-2) prepared in Example-6 of the present application.

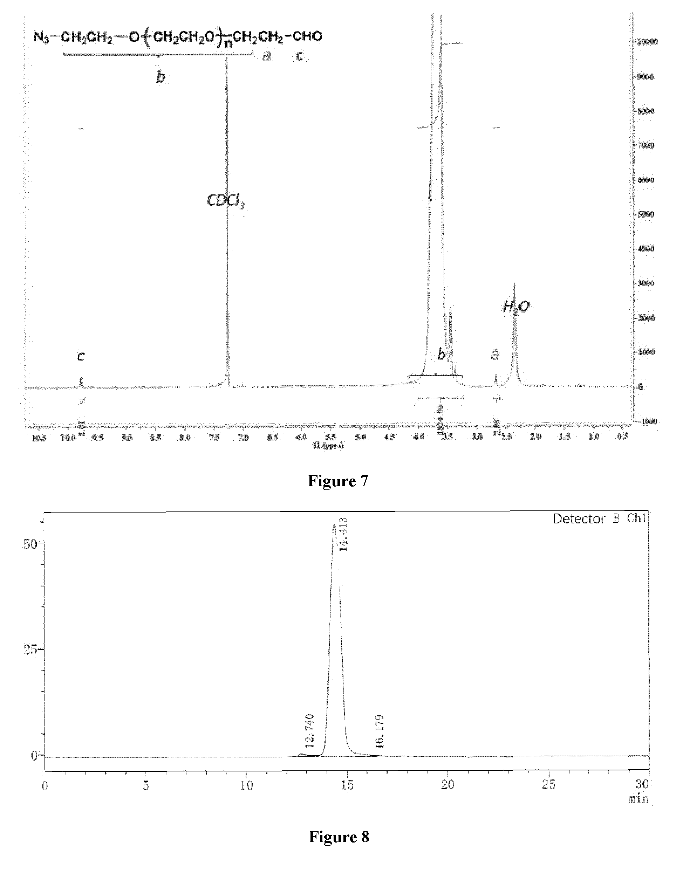

Figure 7 is the NMR spectrum of N₃-PEG-propionaldehyde-20k (P7-2) prepared in Example-7 of the present application.
Figure 8 is the GPC spectrum of mPEG-propionaldehyde-20k (P1-2) prepared in Example-1 of the present application.

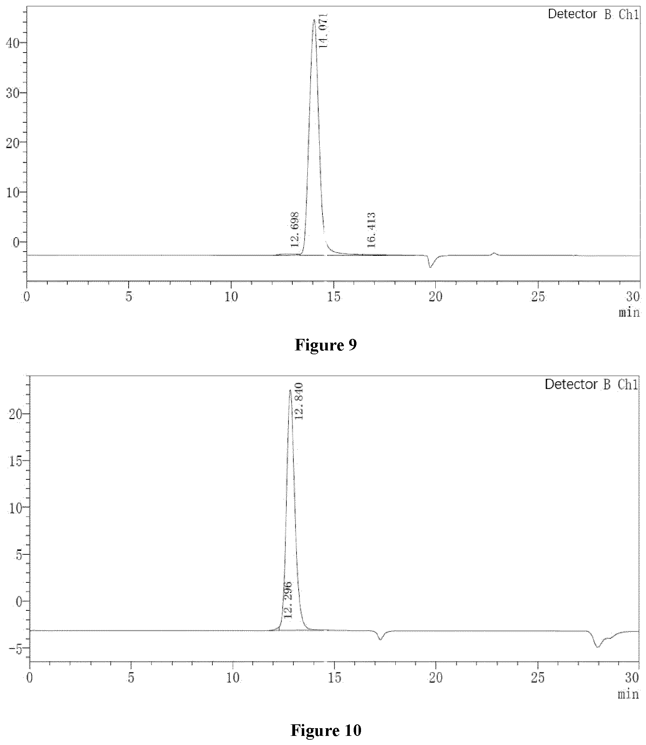

Figure 9 is the GPC spectrum of mPEG-butyraldehyde-20k (P2-2) prepared in Example-2 of the present application.
Figure 10 is the GPC spectrum of PEG-dibutyraldehyde-30k (P3-2) prepared in Example-3 of the present application.

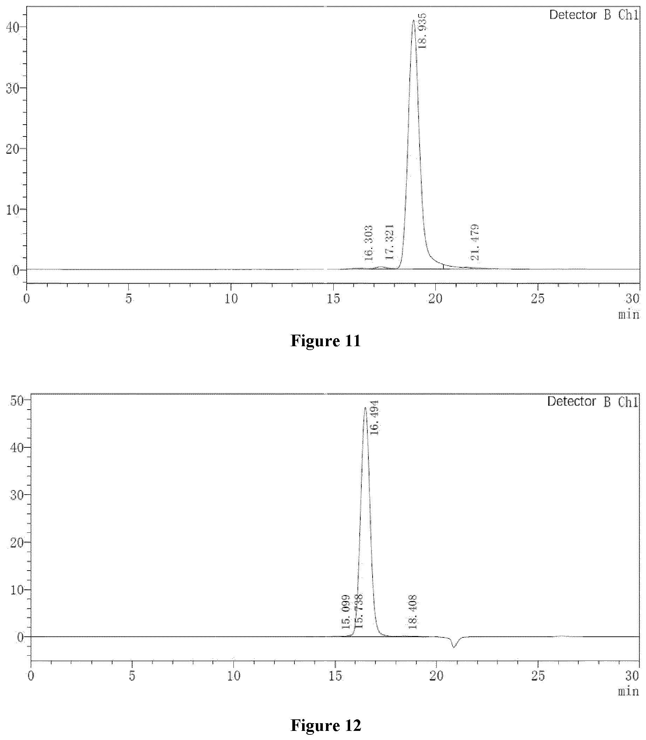

Figure 11 is the GPC spectrum of PEG-dipropionaldehyde-3.4k (P4-2) prepared in Example-4 of the present application.
Figure 12 is the GPC spectrum of 4-arm PEG-propionaldehyde-10k (P5-2) prepared in Example-5 of the present application.

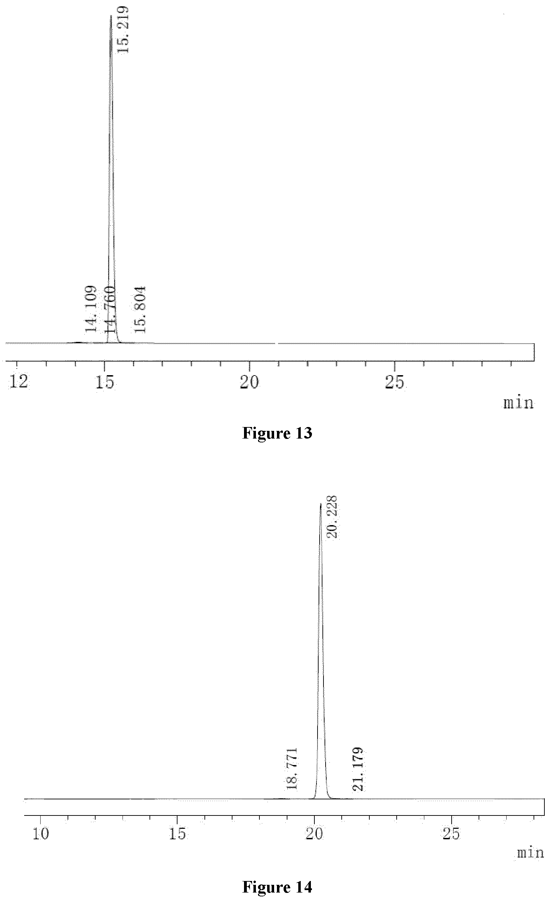

Figure 13 is the HPLC spectrum for testing the terminal substitution rate of mPEG-propionaldehyde-20k (P1-2) prepared in Example-1 of the present application.
Figure 14 is the HPLC spectrum for testing the terminal substitution rate of mPEG-butyraldehyde-20k (P2-2) prepared in Example-2 of the present application.

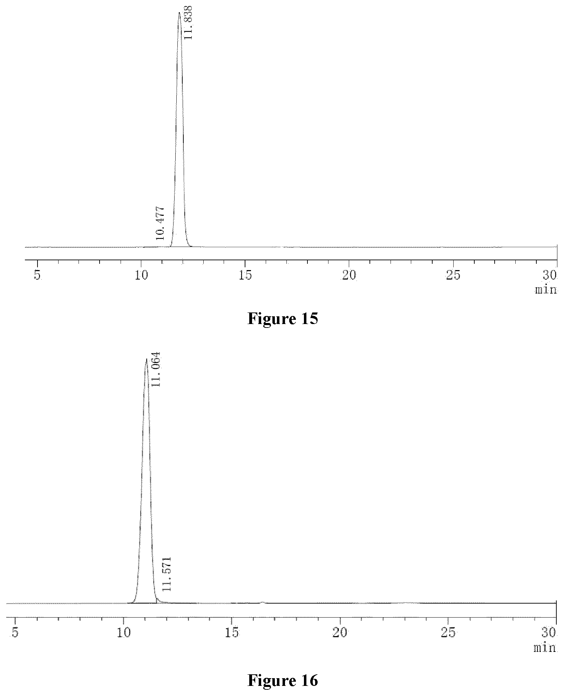

Figure 15 is the HPLC spectrum for testing the terminal substitution rate of PEG-dibutyraldehyde-30k (P3-2) prepared in Example-3 of the present application.
Figure 16 is the HPLC spectrum for testing the terminal substitution rate of PEG-dipropionaldehyde-3.4k (P4-2) prepared in Example-4 of the present application.

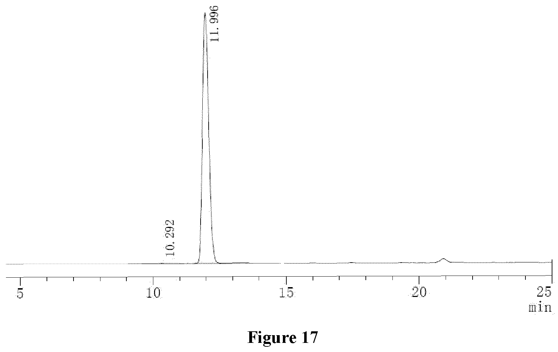

Figure 17 is the HPLC spectrum for testing the terminal substitution rate of 4-arm PEG-propionaldehyde-10k (P5-2) prepared in Example-5 of the present application.

### DETAILED DESCRIPTION

### Description of terms

The specific embodiments of the present application are described in detail. However, it should be understood that the embodiments are only given in an illustrative manner and not in a limiting manner and that various variations and modifications within the scope of the present application will be apparent to those skilled in the art.

In the present application, all technologies and scientific terms used herein have the same meaning as those commonly understood by those of ordinary skill in the art, unless otherwise described. Unless otherwise indicated, the terms have the following meanings.

In the present application, a numerical interval may be indicated by a dash (e.g., 1-6) or a tilde (e.g., 1~6). In the present application, an integer interval represents a group consisting of all integers within the range, including both endpoints, unless otherwise specified. For example, the integer interval 1-6 represents the group consisting of 1, 2, 3, 4, 5, and 6. The numerical interval in the present application includes, but is not limited to, ranges represented by integers, non-integers, percentages, and fractions, and includes both endpoints, unless otherwise specified.

In the present application, "about" and "approximately" generally indicate a range of ±10% for numerical values.

In the present application, when a terminal group of a linking group may be easily confused with a substituent group of the linking group, " 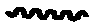 " is used to mark the location where the linking group is connected to other groups. For example, in structural formulas 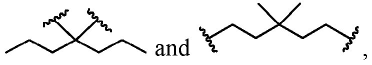 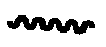 is used to mark the two locations where the divalent linking group is connected to other groups; the two aforementioned structural formulas represent -CH(CH₂CH₂CH₃)₂- and -CH₂CH₂CH(CH₃)₂-CH₂CH₂-, respectively.

In the present application, "molecular weight" represents the molecular mass of a compound. "Average molecular weight" represents the average molecular mass of compound components with the same general formula in a macroscopic substance; unless otherwise specified, "average molecular weight" generally refers to "number average molecular weight" (Mₙ). The number average molecular weight can be used to describe the molecular weight of either polydisperse blocks or substances, or monodisperse blocks or substances. Unless otherwise specified, the measuring unit of "molecular weight" and "average molecular weight" is Dalton (Da). The molecular weight of a polyethylene glycol chain can also be measured by "degree of polymerization" which specifically refers to the number of repeating units (oxyethylene units, EO-units) in a compound molecule. Accordingly, "average degree of polymerization" and preferably "number-average degree of polymerization" are used to characterize the average value and the number average value of the number of repeating units.

In the present application, for polydisperse cases, when a term such as "equal", "same", "equivalent", or "approximately equal" (including other forms of equivalent expression) is used to describe the molecular weight or degree of polymerization of a single compound, or the number-average molecular weight or number-average degree of polymerization of a compound component in macroscopic matter, the term does not impose a strict numerical equality but indicates an approximation or approximate equality in value, unless otherwise specified; the approximation or approximate equality preferably refers to a deviation within ±10%, more preferably a deviation within ±5%, generally based on the preset value. For example, when the molecular weight of mPEG is 5 kDa, it preferably means that the molecular weight value of a single molecule with the general formula is within 4500 to 5500 Da, and the average molecular weight of the corresponding component in the preparation product is 5 kDa, that is, the product with the average molecular weight within 4500 to 5500 Da is the target product.

In the present application, unless otherwise specified, the actions of "selected from" or "preferably selected from" for any two objects are independent from each other. When there are multiple levels of selection or preferred selection, the aforementioned actions for the two objects may occur at the same or different levels. For example, "L_{A} and L_{B} are each independently selected from the group consisting of A, B, and C" includes cases where both L_{A} and L_{B} are A, or where L_{A} is A while L_{B} is B₁ (B₁ is a subset of B). Another example is that "L_{A} is preferably A (level 1 preference), more preferably A₁ to A₃ (level 2 preference), and most preferably A₁₁ to A₁₃ (level 3 preference), while L_{B} is preferably B (level 1 preference), more preferably B₁ to B₃ (level 2 preference), and most preferably B₁₁ to B₁₃ (level 3 preference)" includes cases where the preferable selections are that A is A₁ to A₃ (level 2 preference) while B is B₁₁ to B₁₃ (level 3 preference), or where both A and B are at level 3 preference.

In the present application, phrases such as "each independently", "each independently selected from", and "each independently, preferably selected from" may refer not only to different categories being independently defined by any option within the definitions, but may also include the phrase "at each occurrence" to indicate that the same category at different locations or occurrences is independently defined by any option within the definitions. For example, the two instances of t in formula (2) are, at each occurrence, independently an integer from 1 to 6.

In the present application, when at least two items are enumerated, the term "combination" refers to any combination of two or more of the aforementioned listed items. The quantity of each item within a combination is not limited, which may be zero, one, or greater than one; when the quantity of an item exceeds one, the specific forms of the item may be the same or different. For example, in the context of the statement "the alkali reagent is selected from the group consisting of metal alkoxides, metal hydrides, metal hydroxides, and combinations of any two or more thereof ", a combination of any two listed items may contain two specific metal alkoxides, two specific metal hydrides, or two specific metal hydroxides, which is also may be one metal alkoxide and one metal hydride, one metal alkoxide and one metal hydroxide, or one metal hydride and one metal hydroxide.

In the present application, unless otherwise specified, the term "include" and similar expressions in the specification and claims shall be interpreted in an open and inclusive manner as "include but not be limited to" or "non-restrictively include".

In the present application, terminal substitution rate is also termed terminal activity, referring to the ratio of aldehyde groups as active functional groups present at the ends of PEG. The higher the terminal substitution rate, the higher the degree of aldehyde PEGylation, which makes the PEGylation modifier more effective.

### An embodiment of the present application:

A method for preparing a polyethylene glycol acetal derivative, wherein the structure of the derivative is represented by formula (1) or formula (2): 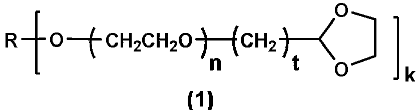 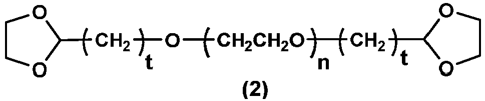
wherein, n is an integer from 20 to 1000;
t is independently an integer from 1 to 6 at each occurrence;
k is an integer from 1 to 8; when k is 1, R is selected from the group consisting of H, -CH₃, -CH₂CH₃, -TBS, -Bn, -(CH₂)ₜₚ-COOH, -(CH₂)ₜₚ-COOtBu and -(CH₂)ₜₚ-N₃; tp is an integer from 1 to 3; when k is an integer from 2 to 8, R is a multivalent branched structure, ranging from divalent to octavalent;
the preparation method comprises:
   dissolving small molecule acetal derivative **I-2** in an organic solvent; activating **I-2** with an alkali reagent; adding polyethylene glycol derivative **I-1** or **I-3**, each of which contains functional group X₁ and is dissolved in an organic solvent; and conducting the reaction for 2 to 24 hours at 20 to 90°C to obtain the polyethylene glycol acetal derivative represented by formula (1) or formula (2); the alkali reagent is sodium hydroxide or potassium hydroxide; wherein, X₁ is independently a p-toluenesulfonyl group or a methanesulfonyl group at each occurrence; X₂ is -OH;

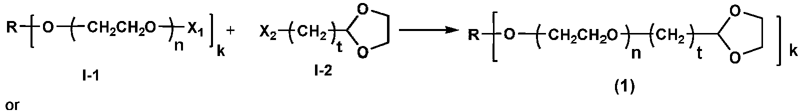

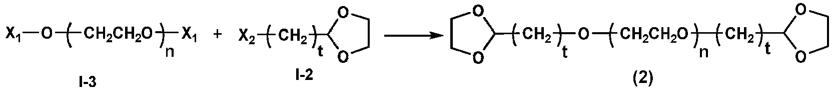

In a specific embodiment of the present application, R is preferably selected from the following cases:
Case 1: k is 1, and R is selected from the group consisting of H, -CH₃, -CH₂CH₃, -TBS, -Bn, -(CH₂)ₜₚ-COOH, -(CH₂)ₜₚ-COOtBu, -(CH₂)ₜₚNH₂, and -(CH₂)ₜₚNHCbz;
Case 2: k is 2, and R is -CH₂CH₂-;
Case 3: k is 3, and R is selected from the group consisting of 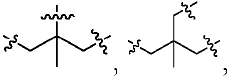 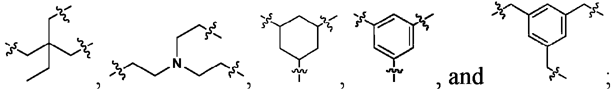
Case 4: k is 4, and R is 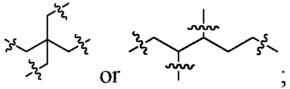
Case 5: k is 6, and R is 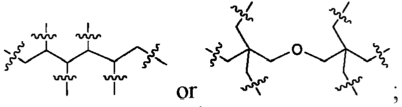
Case 6: k is 8, and R is

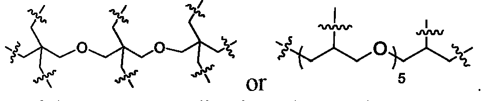

In a specific embodiment of the present application, the number average molecular weight of each polyethylene glycol chain in formula (1) or formula (2) is preferably selected from the group consisting of 900, 1000, 1500, 2000, 2500, 3000, 3350, 3400, 3500, 4000, 5000, 5500, 6000, 6500, 7000, 7500, 8000, 8500, 9000, 9500, 10000, 11000, 15000, 20000, 25000 and 30000 X₁ is a p-toluenesulfonyl group or a methanesulfonyl group, X₂ is -OH, and the alkali reagent is sodium hydroxide or potassium hydroxide;
. In a specific embodiment of the present application, it is preferred that X₁ is a methane sulfonyl , X₂ is -OH, and the preparation method comprises: 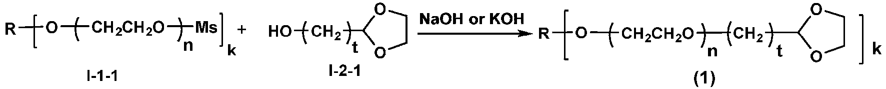 or

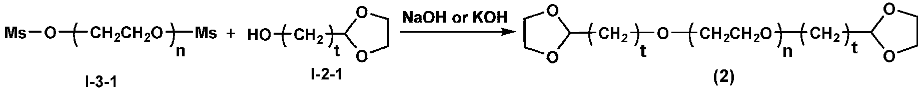

In a specific embodiment of the present application, the organic solvent is not particularly limited, provided that it is capable of dissolving the starting material and the product. The organic solvent is preferably selected from the group consisting of toluene, dichloromethane, chloroform, acetonitrile, dimethylsulfoxide, tetrahydrofuran, *N,N'*-dimethylformamide, *N,N'*-dimethylacetamide, 1,4-dioxane, and combinations of any two or more thereof, more preferably toluene or tetrahydrofuran, and most preferably anhydrous tetrahydrofuran.

In a specific embodiment of the present application, the temperature for the activation treatment with the alkali reagent in the aforementioned embodiments is preferably 20 to 60°C, more preferably 30 to 50°C, and most preferably 40°C; the duration of the activation treatment is preferably 6 to 24 hours, more preferably 8 to 12 hours; the molar ratio of small molecule acetal derivative **I-2** to the alkali reagent is preferably from 1:1 to 2:1; more preferably from 1:1 to 1.5:1, and most preferably 1:1, 1.25:1, or 1.2:1.

In a specific embodiment of the present application, the molar ratio of **I-1** to small molecule acetal derivative **I-2** is preferably from 1:10 to 1:160; when k is 1, the molar ratio of **I-1** to **I-2** is preferably from 1:10 to 1:30; the molar ratio of polyethylene glycol derivative **I-3** containing functional group X₁ to small molecule acetal derivative **I-2** is preferably from 1:20 to 1:100.

In a specific embodiment of the present application, the reaction is preferably conducted under an inert gas, and the inert gas is selected from the group consisting of nitrogen, helium, argon, and any combination thereof.

In a specific embodiment of the present application, the starting material **I-1** or **I-3** for the reaction is added dropwise to **I-2**; the purification process is conducted with precipitation or recrystallization; the precipitating reagent is an ether reagent, selected from an ethyl ether or a methyl tert-butyl ether; the recrystallization reagent is selected from the group consisting of isopropanol, *n*-hexane, and any combination thereof; more preferably, the recrystallization reagent is a mixture of isopropanol and *n*-hexane, and the volume ratio of isopropanol to *n*-hexane is from 2:1 to 5:1.

In a specific embodiment of the present application, the structure of **I-1** is preferably represented by 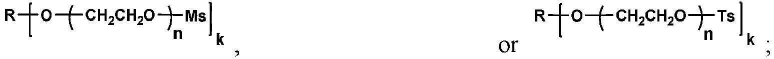 wherein, the definition of R is the same as that described in formula (1). 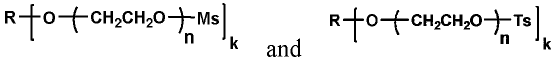 can be obtained through the methanesulfonylation or *p*-toluenesulfonylation of their corresponding alcohols; for example, 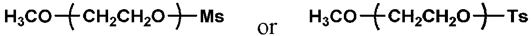 can be obtained through the methanesulfonylation or *p*-toluenesulfonylation of 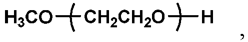 while 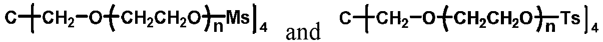 can be obtained through the methanesulfonylation or *p*-toluenesulfonylation of

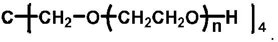

In a specific embodiment of the present application, the structure of **I-1** is preferably selected from the group consisting of

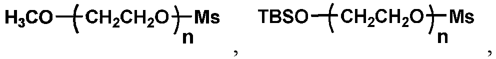

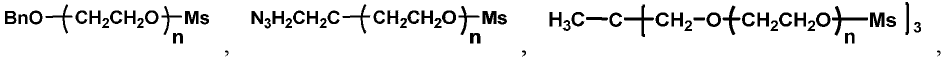

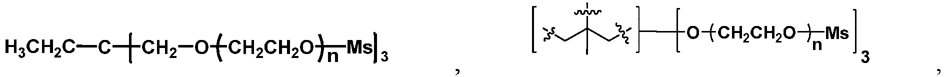

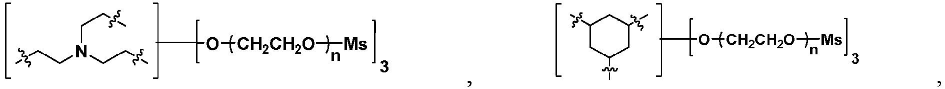

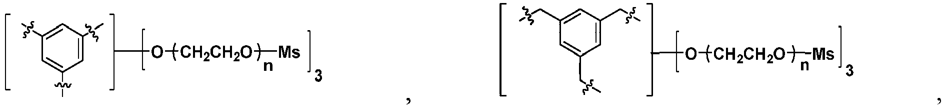

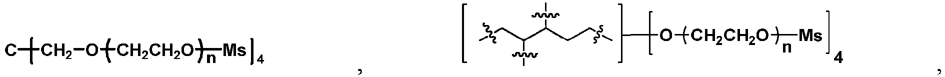

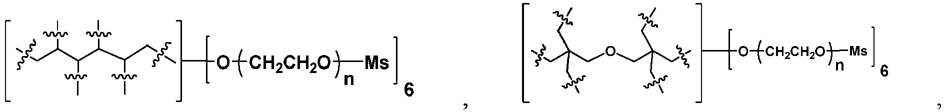

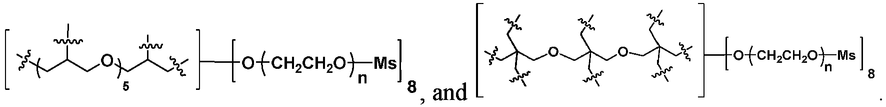

In a specific embodiment of the present application, the structure of **I-3** is preferably 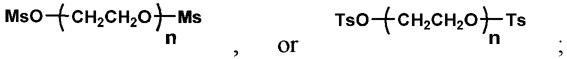 similarly, 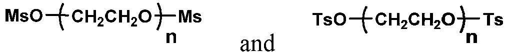 can be obtained through the methanesulfonylation or *p*-toluenesulfonylation of

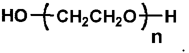

In a specific embodiment of the present application, the structure of **I-2** is preferably selected from the group consisting of

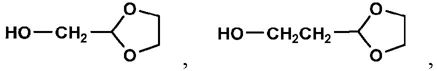

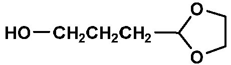

### Another embodiment of the present application:

A method for preparing a polyethylene glycol aldehyde derivative, wherein the method comprises subjecting a polyethylene glycol acetal derivative, which is prepared by any of the aforementioned methods, to acid treatment. In the present application, almost all of the polyethylene glycol acetal derivatives are converted to the corresponding polyethylene glycol aldehyde derivatives, and the ¹HNMR spectra of polyethylene glycol aldehyde derivatives do not exhibit signals of -CH< protons of cyclic acetal groups (chemical shift approximately 4.80 to 5.00) and contain characteristic peaks for aldehyde groups (chemical shift approximately 9.70 to 9.90).

In one specific embodiment of the present application, the acid used in the preparation of the polyethylene glycol aldehyde derivative is selected from the group consisting of hydrochloric acid, trifluoroacetic acid, formic acid, acetic acid and mixtures thereof.

In one specific embodiment of the present application, the structures of the polyethylene glycol aldehydes prepared according to the described method include but are not limited to the following structures:

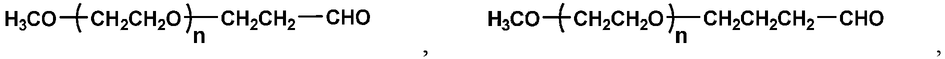

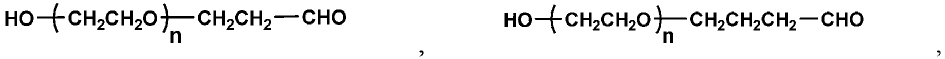

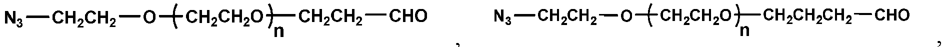

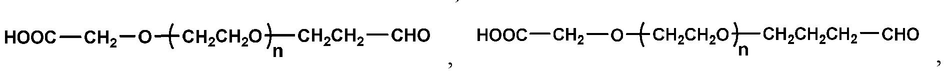

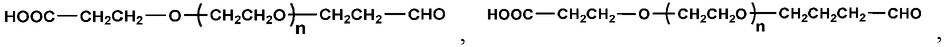

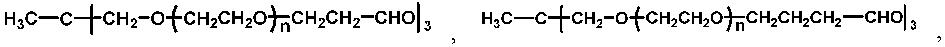

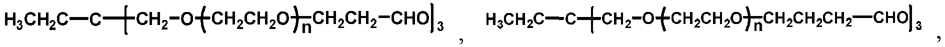

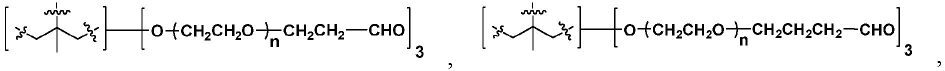

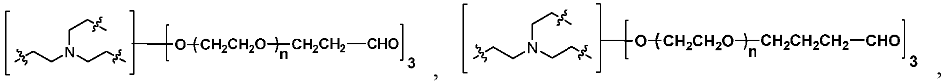

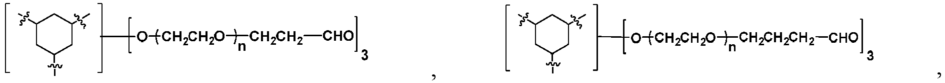

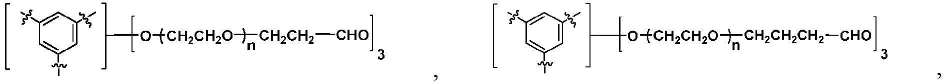

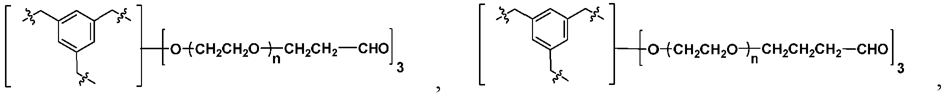

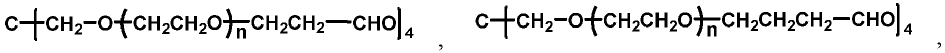

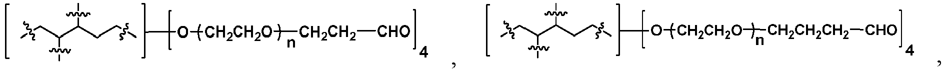

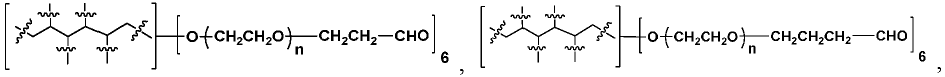

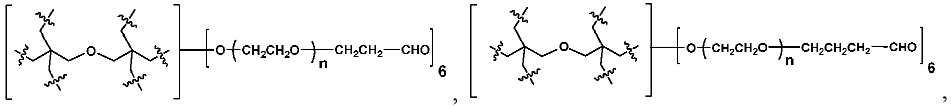

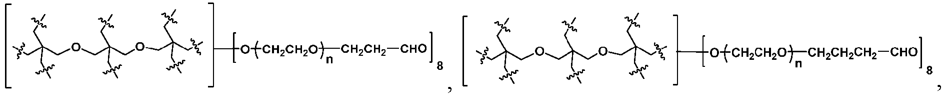

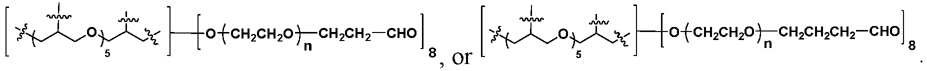

In the present application, the starting materials used in each preparation method can be commercially purchased or synthesized independently.

### Another embodiment of the present application:

A bio-related substance modified with a polyethylene glycol aldehyde derivative prepared according to the aforementioned preparation method; wherein, the bio-related substance is selected from the group consisting of a peptide, a polypeptide, a protein, a polysaccharide, a steroid, a nucleotide, an oligonucleotide, a polynucleotide, and a lipid; more preferably, the bio-related substance contains an active amino group, or, after modification, contains at least one active amino group capable of coupling with the aldehyde group of the polyethylene glycol aldehyde derivative.

The following is a further description with specific examples of the preparation methods of polyethylene glycol acetal derivatives and polyethylene glycol aldehyde derivatives. The specific examples are provided for illustrative purposes only and do not limit the scope of protection of the present application. The intermediates and end products prepared in the present application can be purified by purification methods including but not limited to extraction, recrystallization, adsorption treatment, precipitation, counter precipitation, etc. The structure and molecular weight of the end product may be characterized by characterization methods including but not limited to NMR, electrophoresis, UV-visible spectrophotometry, FTIR, AFM, GPC, HPLC, MALDI-TOF, circular dichroism, etc. In the present application, the structures are preferably characterized by ¹H-NMR, the number average molecular weight (Mn) and polydispersity index (PDI) are preferably determined by gel permeation chromatography (GPC), and the terminal substitution rates are preferably analyzed by high-performance liquid chromatography (HPLC).

### Example-1: Preparation of mPEG-propionaldehyde

### Example-1.1: Preparation of mPEG-propionaldehyde-20k (P1-2)

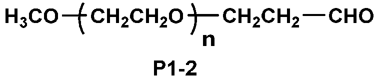

The preparation process is as follows:
Step a: under nitrogen protection, **S1-1** (0.89 g, 7.5 mmol) and anhydrous THF (50 mL) were added to a dry and clean 250 mL round-bottom flask, and NaOH (0.25 g, 6.25 mmol) was added in an ice bath and stirred until dissolved. The reaction was stirred at 40°C for 8 hours. After the reaction was completed, the reaction solution was cooled to room temperature. mPEG-OMs-20k (5.00 g, 0.25 mmol, Mn=20 kDa), dissolved in anhydrous THF (50 mL), was added dropwise to the aforementioned reaction solution. After the addition was completed, the reaction was stirred at 40°C for 8 hours. After the reaction was completed, the reaction solution was cooled to room temperature, and then added with 10% NaCl solution, washed three times with ethyl acetate, and extracted twice with dichloromethane. The organic phases were combined, dried with anhydrous sodium sulfate, filtered, concentrated, and recrystallized with anhydrous isopropanol/*n*-hexane (v/v=3/1) to obtain mPEG-propylal-20k (**P1-1**, 4.66 g, yield: 92.7%). ¹HNMR (CDCl₃, 400MHz) δ (ppm): 1.87-1.99 (-CH₂C***H**₂*CH<, 2H), 3.36-3.39 (-OC***H**₃,* 3H), 3.40-3.80 (-OC***H**₂*C***H**₂*O*-,* PEG-H; -OC***H***₂CH₂CH<, 2H), 3.82-3.98 (>CHOC***H**₂*C***H**₂*O*<,* 4H), 4.93-4.99 (>C***H***CH₂-, 1H).
Step b: **P1-1** prepared in Step a was added to a dry and clean 500 mL round-bottom flask, followed by adding 1 N HCl solution, and then the reaction was stirred in an ice bath for 8 hours. After the reaction was completed, the pH of the solution was adjusted to 6.4±0.2 with sodium bicarbonate, and then the solution was extracted twice with dichloromethane. The organic phases were combined, dried with anhydrous magnesium sulfate, filtered, and recrystallized with the mixture of anhydrous isopropanol and *n*-hexane to obtain mPEG-propionaldehyde-20k (**P1-2**, yield: 98%). The characteristic peak of the acetal group disappeared and the characteristic peak of the aldehyde group 9.76-9.80 (-C***H***O, 1H) appeared in the spectrum of ¹HNMR (CDCl₃). The terminal substitution rate of the aldehyde group in **P1-2** was determined to be 99% by HPLC. The GPC analysis showed that Mn was 20.1 kDa and PDI was 1.03.

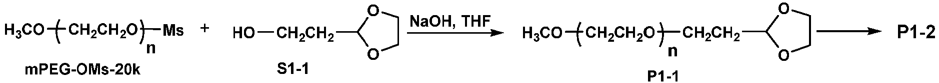

### Example-1.2: Preparation of mPEG-propionaldehyde-2k (P1-4)

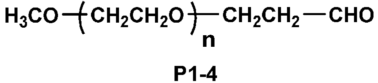

The preparation process is as follows:
Step a: under nitrogen protection, **S1-1** (23.63 g, 200.00 mmol) and anhydrous THF (200 mL) were added to a dry and clean 500 mL round-bottom flask, and NaOH (8.00 g, 200.00 mmol) was added to the solution in an ice bath and stirred until dissolved. The reaction was stirred at 40°C for 8 hours. After the reaction was completed, the reaction solution was cooled to room temperature. mPEG-OMs-20k (20.00 g, 10.00 mmol, Mn=2 kDa), dissolved in anhydrous THF (200 mL), was added dropwise to the aforementioned reaction solution. After the addition was completed, the reaction was stirred at 40°C for 8 hours. After the reaction was completed, the reaction solution was cooled to room temperature, and then added with 10% NaCl solution, washed three times with ethyl acetate, and extracted twice with dichloromethane. The organic phases were combined, dried with anhydrous sodium sulfate, filtered, concentrated, and recrystallized with anhydrous isopropanol/*n*-hexane (v/v=3/1) to obtain mPEG-propylal-2k (**P1-3**, 19.53 g, yield: 93%). ¹HNMR (CDCl₃, 400MHz) δ (ppm): 1.86-1.99 (-CH₂C***H***₂CH<, 2H), 3.35-3.38 (-OC***H**₃,* 3H), 3.40-3.80 (-OC***H**₂*C***H**₂*O*-,* PEG-H; -OC***H***₂CH₂CH₂CH<, 2H), 3.83-3.98 (>CHOC***H**₂*C***H**₂*O*<,* 4H), 4.93-4.99 (>C***H***CH₂-, 1H).
Step b: **P1-3** prepared in Step a was added to a dry and clean 1 L round-bottom flask, followed by adding 1 N HCl solution, and the reaction was stirred in an ice bath for 8 hours. After the reaction was completed, the pH of the solution was adjusted to 6.4±0.2 with sodium bicarbonate, and then the solution was extracted twice with dichloromethane. The organic phases were combined, dried with anhydrous magnesium sulfate, filtered, and recrystallized with the mixture of anhydrous isopropanol and *n*-hexane to obtain mPEG-propionaldehyde-2k (**P1-4,** yield: 98%). The characteristic peak of the acetal group disappeared and the characteristic peak of the aldehyde group 9.76-9.80 (-C***H***O, 1H) appeared in the spectrum of ¹HNMR (CDCl₃, 400MHz). The terminal substitution rate of the aldehyde group in **P1-4** was determined to be 99% by HPLC. The GPC analysis showed that Mn was 2.1 kDa and PDI was 1.03.

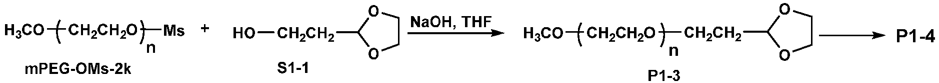

### Example-2: Preparation of mPEG-butyraldehyde-20k (P2-2)

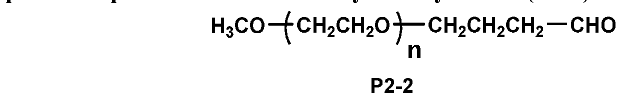

The preparation process is as follows:
Step a: under nitrogen protection, **S2-1** (0.99 g, 7.50 mmol) and anhydrous THF (100 mL) were added to a dry and clean 250 mL round-bottom flask. Then, NaOH (0.20 g, 5.00 mmol) was added in an ice bath and stirred until dissolved. The reaction was stirred at 40°C for 8 hours. After the reaction was completed, the reaction solution was cooled to room temperature. mPEG-OMs-20k (5.00 g, 0.25 mmol, Mn=20 kDa), dissolved in anhydrous THF (50 mL), was added dropwise to the aforementioned reaction solution. After the addition was completed, the reaction was stirred at 55°C for 8 hours. After completion of the reaction, the reaction solution was cooled to room temperature, and then added with 10% NaCl solution, washed three times with ethyl acetate, and extracted twice with dichloromethane. The organic phases were combined, dried with anhydrous sodium sulfate, filtered, concentrated, and recrystallized with anhydrous isopropanol/*n*-hexane (v/v=3/1) to obtain mPEG-butyral-20k (**P2-1**, 4.68 g, yield: 93.1%). ¹HNMR (CDCl₃, 400MHz) δ (ppm): 1.73-1.82 (-CH₂C***H***₂C***H***₂CH<, 4H), 3.32-3.37 (-OC***H**₃,* 3H), 3.38-3.80 (-OC***H**₂*C***H**₂*O*-,* PEG-H; -OC***H***₂CH₂CH₂CH<, 2H), 3.84-4.09 (>CHOC***H**₂*C***H**₂*O*<,* 4H), 4.82-4.90 (>C***H***CH₂-, 1H).
Step b: **P2-1** prepared in Step a was added to a dry and clean 500 mL round-bottom flask, followed by adding 1 N HCl solution, and the reaction was stirred in an ice bath for 8 hours. After the reaction was completed, the pH of the solution was adjusted to 6.4±0.2 with sodium bicarbonate, and then the solution was extracted twice with dichloromethane. The organic phases were combined, dried with anhydrous magnesium sulfate, filtered, and recrystallized with the mixture of anhydrous isopropanol and *n*-hexane to obtain mPEG-butyraldehyde-20k (**P2-2**, yield: 97%). The characteristic peak of the acetal group disappeared and the characteristic peak of the aldehyde group 9.75-9.78 (-C***H***O, 1H) appeared in the spectrum of ¹HNMR (CDCl₃, 400MHz). The terminal substitution rate of the aldehyde group in **P2-2** was determined to be 99% by HPLC. The GPC analysis showed that Mn was 20.1 kDa and PDI was 1.03.

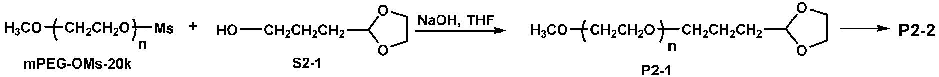

### Example-3: Preparation of PEG-30k-dibutyraldehyde (P3-2)

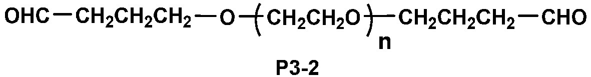

The preparation process is as follows:
Step a: **S2-1** (4.36 g, 33.00 mmol) and anhydrous THF (50 mL) were added to a dry and clean 1 L round-bottom flask. Then, NaOH (4.26 g, 106.00 mmol) was added in an ice bath and stirred until dissolved. The reaction was stirred at 40°C for 8 hours. After the reaction was completed, the reaction solution was cooled to room temperature. MsO-PEG-OMs-30k (9.90 g, 0.33 mmol, Mn=30 kDa), dissolved in anhydrous THF (100 mL), was added dropwise to the aforementioned reaction solution. After the addition was completed, the reaction was stirred at 55°C for 8 hours. After completion of the reaction, the reaction solution was cooled to room temperature, and then added with 10% NaCl solution, washed three times with ethyl acetate, and extracted twice with dichloromethane. The organic phases were combined, dried with anhydrous sodium sulfate, filtered, concentrated, and recrystallized with anhydrous isopropanol/*n*-hexane (v/v=3/1) to obtain PEG-dibutyral-30k (**P3-1,** 9.31 g, yield: 93.8%). ¹HNMR (d₆-DMSO, 400MHz) δ (ppm): 1.50-1.65 (-OCH₂C***H***₂C***H***₂CH<, 8H), 3.35-3.65 (-OC***H**₂C**H**₂*O*-,* PEG-H; -OCH₂CH₂OC***H***₂-, 4H), 3.70-3.90 (>CHOC***H**₂*C***H**₂*O*<,* 8H), 4.75-4.85 (>C***H***CH₂-, 2H).
Step b: **P3-1** prepared in Step a was added to a dry and clean 2 L round-bottom flask, followed by adding 1 N HCl solution, and the reaction was stirred in an ice bath for 8 hours. After completion of the reaction, the pH of the solution was adjusted to 6.4±0.2 with sodium bicarbonate, and then the solution was extracted twice with dichloromethane. The organic phases were combined, dried with anhydrous magnesium sulfate, filtered, and recrystallized with the mixture of anhydrous isopropanol and *n*-hexane to obtain PEG-dibutyraldehyde-30k (**P3-2**, yield: 97%). The characteristic peak of the acetal group disappeared and the characteristic peak of the aldehyde group 9.79-9.83 (-C***H***O, 2H) appeared in the spectrum of ¹HNMR (d₆-DMSO, 400MHz). The terminal substitution rate of the aldehyde group in **P3-2** was determined to be 99% by HPLC. The GPC analysis showed that Mn was 30.2 kDa and PDI was 1.04.

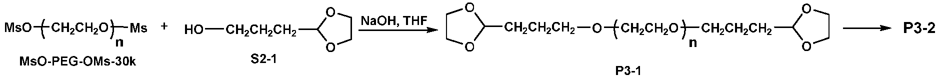

### Example-4: Preparation of PEG-dipropionaldehyde-3.4k (P4-2)

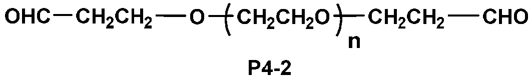

The preparation process is as follows:
Step a: under nitrogen protection, **S1-1** (93.56 g, 792.00 mmol) and anhydrous THF (300 mL) were added to a dry and clean 1 L round-bottom flask. Then, NaOH (21.12 g, 528.00 mmol) was added in an ice bath and stirred until dissolved. The reaction was stirred at 40°C for 8 hours. After completion of the reaction, the reaction solution was cooled to room temperature. MsO-PEG-OMs-3.4k (29.92 g, 8.80 mmol, Mn=3.4 kDa), dissolved in anhydrous THF (300 mL), was added dropwise to the aforementioned reaction solution. After the addition was completed, the reaction was stirred at 40°C for 8 hours. After the reaction was completed, the reaction solution was cooled to room temperature, and then added with 10% NaCl solution, washed three times with ethyl acetate, and extracted twice with dichloromethane. The organic phases were combined, dried with anhydrous sodium sulfate, filtered, concentrated, and recrystallized with anhydrous isopropanol/n-hexane (v/v=3/1) to obtain PEG-dipropylal-3.4k (**P4-1**, 28.14 g, yield: 92.2%). ¹HNMR (CDCl₃, 400MHz) δ (ppm): 1.86-1.99 (-CH₂C***H***₂CH<, 4H), 3.38-3.80 (-OC***H**₂*C***H**₂*O*-,* PEG-H; -OC***H***₂CH₂CH<, 4H), 3.83-3.98 (>CHOC***H***₂C***H***₂O<, 8H), 4.93-4.99 (>C***H***CH₂-, 2H).
Step b: **P4-1** prepared in Step a was added to a dry and clean 2 L round-bottom flask, followed by adding 1 N HCl solution, and the reaction was stirred in an ice bath for 8 hours. After the reaction was completed, the pH of the solution was adjusted to 6.4±0.2 with sodium bicarbonate, and then the solution was extracted twice with dichloromethane. The organic phases were combined, dried with anhydrous magnesium sulfate, filtered, and recrystallized with the mixture of anhydrous isopropanol and *n*-hexane to obtain PEG-dipropionaldehyde-3.4k (**P4-2**, yield: 97%). The characteristic peak of the acetal group disappeared and the characteristic peak of the aldehyde group 9.74-9.85 (-C***H***O, 2H) appeared in the spectrum of ¹HNMR (CDCl₃, 400MHz). The terminal substitution rate of the aldehyde group in **P4-2** was determined to be 99% by HPLC. The GPC analysis showed that Mn was 3.6 kDa and PDI was 1.03.

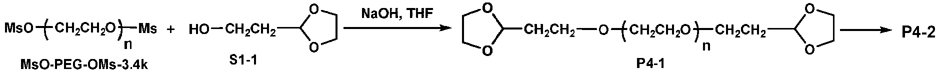

### Comparative example-5: Preparation of 4-arm PEG-propionaldehyde

### Example-5.1: Preparation of 4-arm PEG-propionaldehyde 4-arm-PEG-pALD-10k (P5-2)

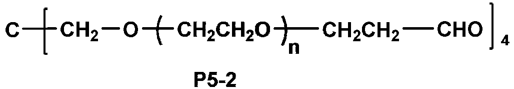

The preparation process is as follows:
Step a: under nitrogen protection, **4-arm-PEG-OH** (25.00 g, 2.50 mmol, Mn=10 kDa) was added to a dry and clean 2 L round-bottom flask, and water was removed through azeotropic distillation with toluene. **NaH** (16.00 g, 400.00 mmol), dissolved in anhydrous THF (1 L), was added to the aforementioned 4-arm-PEG-OH solution. The reaction temperature was raised to 85°C and the reaction was refluxed for 8 hours. After the reaction was completed, the reaction solution was cooled to room temperature, and then added with NaI (1.50 g, 10.00 mmol) and **S5-1** (72.41 g, 400.00 mmol). The reaction temperature was raised to 85°C and the reaction was refluxed for 8 hours. After the reaction was completed, the mixture was placed in an ice bath, and an aqueous solution of ammonium chloride was slowly added dropwise to quench the reaction. Then, THF was removed by concentration, followed by adding 10% NaCl solution. The mixture was washed three times with ethyl acetate and then extracted twice with dichloromethane. The organic phases were combined, dried with anhydrous sodium sulfate, filtered, concentrated, and recrystallized with anhydrous isopropanol/n-hexane (v/v=3/1) to obtain 4-arm-PEG-propylal-10k (**P5-1**, 23.59 g, yield: 90.7%). ¹HNMR (CDCl₃, 400MHz) δ (ppm): 1.85-1.97 (-CH₂C***H**₂*CH<, 8H), 3.40-3.80 (-OC***H**₂*C***H**₂*O*-,* PEG-H; -OC*H*₂CH₂CH<, 8H; -CC***H***₂-, 8H), 3.83-3.98 (>CHOC***H**₂*C***H**₂*O<, 16H), 4.91-4.97 (>C***H***CH₂-, 4H).
Step b: **P5-1** prepared in Step a was added to a dry and clean 2 L round-bottom flask, followed by adding 1 N HCl solution, and the reaction was stirred in an ice bath for 8 hours. After the reaction was completed, the pH of the solution was adjusted to 6.4±0.2 with sodium bicarbonate, and then the solution was extracted twice with dichloromethane. The organic phases were combined, dried with anhydrous magnesium sulfate, filtered, and recrystallized with the mixture of anhydrous isopropanol and *n*-hexane to obtain 4-arm-PEG-propionaldehyde-10k (**P5-2**, yield: 97%). The characteristic peak of the acetal group disappeared and the characteristic peak of the aldehyde group 9.76-9.82 (-C***H***O, 4H) appeared in the spectrum of ¹HNMR (CDCl₃, 400MHz). The terminal substitution rate of aldehyde group in **P5-2** was determined to be 97% by HPLC. The GPC analysis showed that Mn was 10.4 kDa and PDI was 1.04.

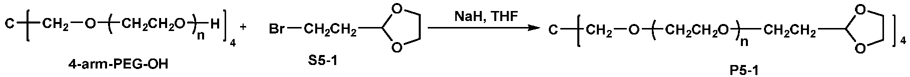

### Example-5.2: Preparation of 4-arm PEG-propionaldehyde 4-arm-PEG-pALD-20k

Following the procedures in Example-5.1, the starting material 4-arm-PEG-OH-10k was replaced with 4-arm-PEG-OH-20k to obtain 4-arm-PEG-propylal-20k **(P5-3)** and 4-arm-PEG-propanal-20k **(P5-4).** The structures were also verified by ¹HNMR, GPC, and HPLC.

### Example-6: Preparation of hydroxy-PEG-propionaldehyde-20k (P6-2)

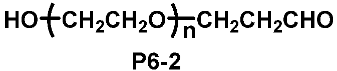

The preparation process is as follows:
Step a: under nitrogen protection, S1-1 (0.89 g, 7.50 mmol) and anhydrous THF (50 mL) were added to a dry and clean 250 mL round-bottom flask. Then, NaOH (0.20 g, 5.00 mmol) was added in an ice bath and stirred until dissolved. The reaction was stirred at 40°C for 8 hours. After the reaction was completed, the reaction solution was cooled to room temperature. TBSO-PEG-OMs-20k (5.00 g, 0.25 mmol), dissolved in anhydrous THF (50 mL), was added dropwise to the aforementioned reaction solution. After the addition was complete, the reaction was stirred at 40°C for 8 hours. After the reaction was completed, the reaction solution was cooled to room temperature, and then added with 10% NaCl solution, washed three times with ethyl acetate, and extracted twice with dichloromethane. The organic phases were combined, dried with anhydrous sodium sulfate, filtered, concentrated, and recrystallized with anhydrous isopropanol/*n*-hexane (v/v=3/1) to obtain TBSO-PEG-propylal-20k (**P6-1**, 4.65 g, yield: 93.0%). ¹HNMR (CDCl₃, 400MHz) δ (ppm): 0.21 (-Si(C***H***₃)₂-, 6H), 0.98 (-SiC(C***H***₃)₃, 9H), 1.75-1.84 (-CH₂C***H***₂CH<, 2H), 3.35-3.72 (-OC***H**₂*C***H**₂*O*-,* PEG-H; -OC***H***₂CH₂CH₂CH<, 2H), 3.71-3.90 (>CHOC***H**₂*C***H**₂*O<, 4H), 4.80-4.90 (>C***H***CH₂-, 1H).
Step b: **P6-1** prepared in Step a was added to a dry and clean 500 mL round-bottom flask and dissolved with a mixed solution of tetrahydrofuran/acetic acid. TBAF was added in an ice bath. The reaction was conducted at room temperature for 8 hours. After extraction and concentration, 1 N HCl solution was added to the reaction solution and the reaction was stirred in an ice bath for 8 hours. After completion of the reaction, the pH of the solution was adjusted to 6.4±0.2 with sodium bicarbonate, and then the solution was extracted twice with dichloromethane. The organic phases were combined, dried with anhydrous magnesium sulfate, filtered, and recrystallized with the mixture of anhydrous isopropanol and *n*-hexane to obtain hydroxy-PEG-propionaldehyde-20k (**P6-2**, yield: 95%). The characteristic peaks of the acetal and TBS groups disappeared and the characteristic peak of the aldehyde group 9.73-9.80 (-C***H***O, 1H) appeared in the spectrum of ¹HNMR (CDCl₃, 400MHz). The terminal substitution rate of the aldehyde group in **P6-2** was determined to be 97% by HPLC. The GPC analysis showed that Mn was 20.1 kDa and PDI was 1.04.

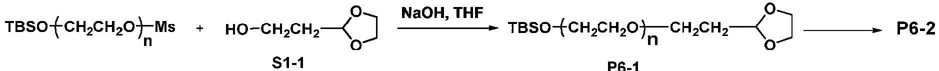

### Example-7: Preparation of N₃-PEG-propionaldehyde-20k (P7-2)

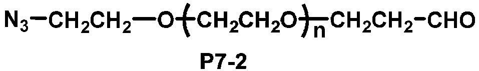

The preparation process is as follows:
Step a: under nitrogen protection, **S1-1** (0.89 g, 7.50 mmol) and anhydrous THF (50 mL) were added to a dry and clean 250 mL round-bottom flask. Then, NaOH (0.20 g, 5.00 mmol) was added under in ice bath and stirred until dissolved, and the reaction was stirred at 40°C for 8 hours. After the reaction was completed, the reaction solution was cooled to room temperature. N₃-PEG-OMs-20k (5.00 g, 0.25 mmol), dissolved in anhydrous THF (50 mL), was added dropwise to the aforementioned reaction solution. After the addition was completed, the reaction was stirred at 40°C for 8 hours. After completion of the reaction, the reaction solution was cooled to room temperature, and then added with 10% NaCl solution, washed three times with ethyl acetate, and extracted twice with dichloromethane. The organic phases were combined, dried with anhydrous sodium sulfate, filtered, concentrated, and recrystallized with anhydrous isopropanol/*n*-hexane (v/v=3/1) to obtain N₃-PEG-propylal-20k (**P7-1**, 4.65 g, yield: 93.0%). ¹HNMR (CDCl₃, 400MHz) δ (ppm): 1.75-1.84 (-CH₂C***H***₂CH<, 2H), 3.35-3.72 (-OC***H**₂*C***H**₂*O*-,* PEG-H; -OC***H***₂CH₂CH₂CH<, 2H; -OC***H***₂C***H***₂N₃, 4H), 3.71-3.90 (>CHOC***H**₂*C***H**₂*O<, 4H), 4.80-4.90 (>C***H***CH₂-, 1H).
Step b: **P7-1** prepared in Step a and 1 N HCl solution were successively added to a dry and clean 500 mL round-bottom flask, and the mixture was stirred in an ice bath for 8 hours. After the reaction was completed, the pH of the solution was adjusted to 6.4±0.2 with sodium bicarbonate, and then the solution was extracted twice with dichloromethane. The organic phases were combined, dried with anhydrous magnesium sulfate, filtered, and recrystallized with the mixture of anhydrous isopropanol and *n*-hexane to obtain N₃-PEG-propionaldehyde-20k (**P7-2**, yield: 95%). The characteristic peak of the acetal group disappeared and the characteristic peak of the aldehyde group 9.73-9.80 (-C***H***O, 1H) appeared in the spectrum of ¹HNMR (CDCl₃, 400MHz). The terminal substitution rate of the aldehyde group in **P7-2** was determined to be 99% by HPLC. The GPC analysis showed that Mn was 20 1 kDa and PDT was 1 03

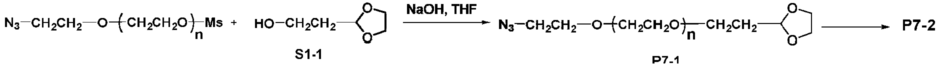

### Comparative example-8:

### : Preparation method 2 of mPEG-propionaldehyde-20k (P1-2)

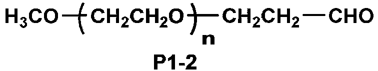

The preparation process is as follows:
Step a: under nitrogen protection, **mPEG-OH** (5.00 g, 0.25 mmol, Mn=20 kDa) was added to a dry and clean 2 L round-bottom flask, and water was removed through azeotropic distillation with toluene. **NaH** (0.20 g, 5.00 mmol), dissolved in anhydrous THF (50 mL), was added to the aforementioned **mPEG-OH** solution in an ice bath. The temperature was raised to 85°C and the reaction was refluxed for 8 hours. After the reaction was completed, **S5-1** (1.36 g, 7.50 mmol) was added to the reaction solution at room temperature. The temperature was raised to 85°C and the reaction was refluxed for 8 hours. After completion of the reaction, the mixture was placed in an ice bath, and an aqueous solution of ammonium chloride was slowly added dropwise to quench the reaction. The solution was concentrated to remove THF and then added with 10% NaCl solution, washed three times with ethyl acetate, and extracted twice with dichloromethane. The organic phases were combined, dried with anhydrous sodium sulfate, filtered, concentrated, and recrystallized with anhydrous isopropanol/*n*-hexane (v/v=3/1) to obtain mPEG-propylal-20k **(P1-1,** 2.30 g, yield: 88.5%). The structure was also characterized by NMR.
Step b: **P1-1** prepared in Step a and 1 N HCl solution was successively added to a dry and clean 2 L round-bottom flask, and the reaction was stirred in an ice bath for 8 hours. After completion of the reaction, the pH of the solution was adjusted to 6.4±0.2 with sodium bicarbonate, and then the solution was extracted twice with dichloromethane. The organic phases were combined, dried with anhydrous magnesium sulfate, filtered, and recrystallized with the mixture of anhydrous isopropanol and *n*-hexane to obtain mPEG-propionaldehyde-20k **(P1-2,** yield: 97%). The characteristic peak of the acetal group disappeared and the characteristic peak of the aldehyde group 9.76-9.82 (-C***H***O, 1H) appeared in the spectrum of ¹HNMR (CDCl₃, 400MHz). The terminal substitution rate of the aldehyde group in **P1-2** was determined to be 95% by HPLC.

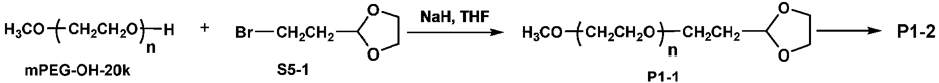

### Comparative example-9:

### : Preparation method 3 of mPEG-propionaldehyde-20k (P1-2)

The alkali reagent NaH in Example-8 was replaced with NaOH, while the other steps remained the same as those in Example-8, resulting in a final yield of **P1-2** of 84%. The terminal substitution rate of the aldehyde group in **P1-2** was determined to be 99% by HPLC.

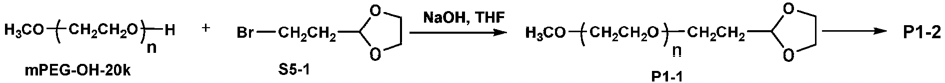

### Comparative example-10:

### Preparation method 4 of mPEG-propionaldehyde-20k (P1-2)

The preparation process is as follows:
The small molecule cyclic acetal starting material **S5-1** in Example-8 was replaced with the small molecule linear acetal starting material **S10-1,** while the other steps remained the same as those in Example-8, resulting in a final yield of **P1-2** of about 83%. The terminal substitution rate of the aldehyde group in **P1-2** was determined to be 85% by HPLC.

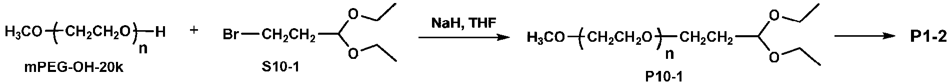

### Comparative example-11:

### Preparation method 5 of mPEG-propionaldehyde-20k (P1-2)

The preparation process is as follows:
The small molecule cyclic acetal starting material **S1-1** in Example-1.1 was replaced with the small molecule linear acetal starting material **S11-1,** while the other steps remained the same as those in Example-1.1, resulting in a final yield of P1-2 of about 87%. The terminal substitution rate of the aldehyde group in **P1-2** was determined to be 87% by HPLC.

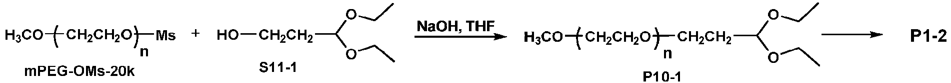

### Example-12: Preparation method 6 of mPEG-propionaldehyde-20k (P1-2)

The starting material **mPEG-OMs-20k** in Example-1.1 was not added dropwise but directly added as a solid, while the other steps remained the same as those in Example-1.1, resulting in a final yield of **P1-2** of about 82%. The terminal substitution rate of the aldehyde group in **P1-2** was determined to be 91% by HPLC.

### Example-13: Preparation method 7 of mPEG-propionaldehyde-20k (P1-2)

The recrystallization procedure in Step a of Example-1.1 was replaced with pulping and precipitation with an ether reagent (t-butyl methyl ether). Specifically, the crude product was dissolved in a small amount of DCM, and the solution was added dropwise to the ether reagent (where the volume of the ether reagent was 20 times that of DCM). All other steps remained the same as those in Example-1.1, resulting in a final yield of **P1-2** of about 87%. The terminal substitution rate of the aldehyde group in **P1-2** was determined to be 89% by HPLC.

### Example-14: Preparation method 8 of mPEG-propionaldehyde-20k (P1-2)

The recrystallization procedure in Step a of Example-1.1 was replaced with recrystallization with isopropanol, while the other steps remained the same as those in Example-1.1, resulting in a final yield of **P1-2** of about 89%. The terminal substitution rate of the aldehyde group in **P1-2** was determined to be 93% by HPLC.

The above Example-1.1 and Example-8 to 14 are different preparation methods of mPEG-propionaldehyde-20k **(P1-2),** while Example-9 to 14 are comparative methods. The comparisons of each method are shown in Tables 1 and 2 below:

**Table 1**

| **Example** | **Acetal Reagent** | **Metallic Alkali** | **Final Yield (%)** | **Terminal Substitution Rate (%)** |
|---|---|---|---|---|
| 1.1 | 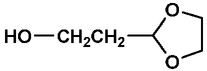 | NaOH | 91 | 99 |
| 8 | 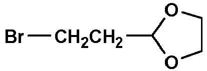 | NaH | 86 | 95 |
| 9 | 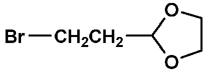 | NaOH | 84 | 38 |
| 10 | 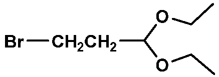 | NaH | 83 | 85 |
| 11 | 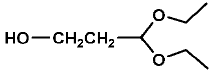 | NaOH | 87 | 87 |

**Table 2**

| **Example** | **Precipitation or Recrystallization** | **Feeding Method of PEG Starting Material** | **Final Yield (%)** | **Terminal Substitution Rate (%)** |
|---|---|---|---|---|
| 1.1 | recrystallization with anhydrous isopropanol/n-hexane (v/v=3/1) | dropwise | 91 | 99 |
| 12 | recrystallization with anhydrous isopropanol/*n*-hexane (v/v=3/1) | non-dropwise | 82 | 91 |
| 13 | precipitation with *t*-butyl methyl ether | dropwise | 87 | 89 |
| 14 | recrystallization with anhydrous isopropanol | dropwise | 89 | 93 |

As shown in Tables 1 and 2 above, there are differences in yield and terminal functional group substitution rate among the various preparation methods, with the difference in terminal substitution rate being more significant. Those skilled in the art know that the terminal substitution rate is more important to the quality of macromolecular products, and the products with a higher terminal substitution rate generally exhibit greater purity.

According to Table 1, in the preparation of polyethylene glycol aldehyde derivatives, the types of small molecule acetal reagents and alkali reagents have a significant impact on the yield and quality of the product. Firstly, a comparison of Examples-1.1, 8, 9 and Examples-10, 11 shows that the use of small molecule cyclic acetal reagents results in a higher yield and a higher terminal substitution rate compared to small molecule linear acetal reagents, which is because the cyclic acetal group is more stable than the linear acetal group and therefore fewer side reactions are expected during the preparation process. Secondly, a comparison of Examples-1.1, 8, and 9 shows that different small molecule acetal reagents correspond to different preferable metallic alkali reagents, wherein NaOH in Example-1.1 is used for dehydrogenation activation of small molecule acetal, NaH in Example-8 and NaOH in Example-9 are used for dehydrogenation activation of the hydrogen in mPEG-OH. The dehydrogenation activation of mPEG-OH is more effective with NaH than with NaOH, resulting in a terminal aldehyde group substitution rate of 95% for the product obtained with NaH, compared to only 38% for the product obtained with NaOH.

According to Table 2, the method of adding starting materials and the post-treatment process have a significant impact on the yield and quality of the products. Firstly, comparing Example-1.1 with Example-12, it can be seen that when the polyethylene glycol starting material is added to the metallic alkali-activated small molecule acetal derivatives, the yield from dropwise addition is higher than that of direct pouring, and the terminal functional group substitution rate of the product is also higher. This may be due to the fact that the starting material added in the dropwise manner can immediately react with the activated other reactant upon addition, so that the reaction is more complete and more time-efficient. Secondly, a comparison of Examples-1.1, 13 and 14 shows that the yield from recrystallization is higher and the product quality is better than that from the precipitation method in post-treatment. Additionally, the selection of the recrystallization reagent also affects the yield and quality of the product, and the use of the mixture of isopropanol and *n*-hexane for the recrystallization gives a higher yield and higher substitution rate of terminal functional groups in the product.

In summary, compared with the comparative Examples 9 to 14, the product obtained by the preparation method of the present application (Example-1.1) has a higher yield, greater purity, and better quality (including a higher substitution rate of the terminal aldehyde group).

## Claims

1. A method for preparing a polyethylene glycol acetal derivative, wherein the structure of the derivative is represented by formula (1) or formula (2): 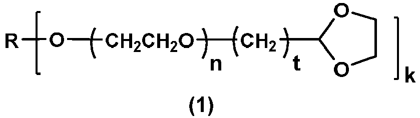 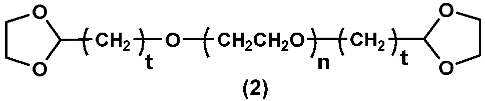
wherein, n is an integer from 20 to 1000;
t is independently an integer from 1 to 6 at each occurrence;
k is an integer from 1 to 8; when k is 1, R is selected from the group consisting of H, - CH₃, -CH₂CH₃, -TBS, -Bn, -(CH₂)ₜₚ-COOH, -(CH₂)ₜₚ-COOtBu, -(CH₂)ₜₚ-N₃; tp is an integer from 1 to 3; when k is an integer from 2 to 8, R is a multivalent branched structure, ranging from divalent to octavalent;
the preparation method comprises:
dissolving small molecule acetal derivative **I-2** in an organic solvent; activating **I-2** with an alkali reagent; adding polyethylene glycol derivative **I-1** or **I-3**, each of which contains functional group X₁ and dissolving in an organic solvent; and then conducting the reaction for 2 to 24 hours at 20 to 90°C to obtain the polyethylene glycol acetal derivative represented by formula (1) or formula (2); the alkali reagent is sodium hydroxide or potassium hydroxide;
wherein, X₁ is independently a *p*-toluenesulfonyl group or a methanesulfonyl group at each occurrence; X₂ is -OH; 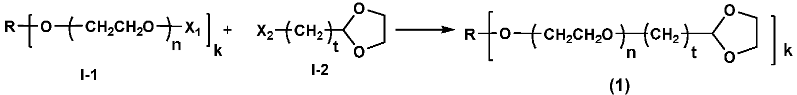 or

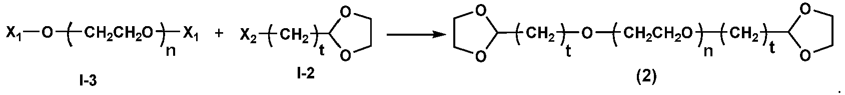

2. The method for preparing polyethylene glycol acetal derivative according to claim **1,** wherein R is selected from the group consisting of H, -CH₃, -CH₂CH₃, -Bn, -(CH₂)ₜₚ-COOH, - (CH₂)ₜₚ-COOtBu, -(CH₂)ₜₚNH₂, -(CH₂)ₜₚNHCbz, -CH₂CH₂-,

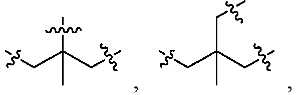

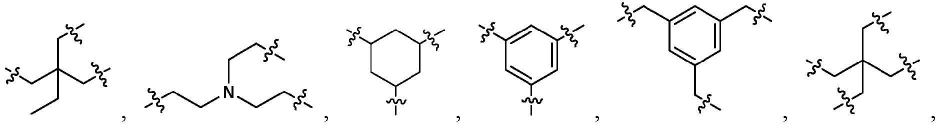

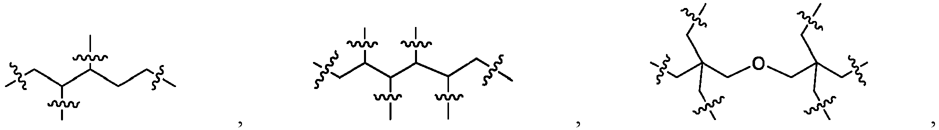

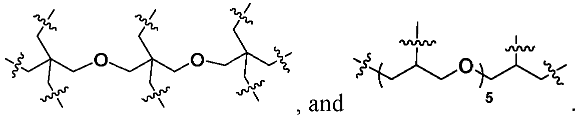

3. The method for preparing polyethylene glycol acetal derivative according to claim 1, wherein the number average molecular weight of each polyethylene glycol chain is selected from the group consisting of 900, 1000, 1500, 2000, 2500, 3000, 3350, 3400, 3500, 4000, 5000, 5500, 6000, 6500, 7000, 7500, 8000, 8500, 9000, 9500, 10000, 11000, 15000, 20000, 25000, and 30000, wherein the number average molecular weight is determined by gel permeation chromatography (GPC).

4. The method for preparing polyethylene glycol acetal derivative according to claim **1,** wherein when X₁ is a methanesulfonyl group, X₂ is -OH, and the preparation method comprises: 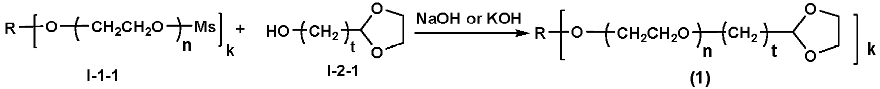 or

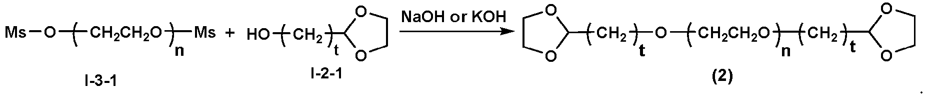

5. The method for preparing polyethylene glycol acetal derivative according to claim **1,** wherein the organic solvent is selected from the group consisting of toluene, dichloromethane, chloroform, acetonitrile, dimethylsulfoxide, tetrahydrofuran, *N,N'*-dimethylformamide, *N,N'-*dimethylacetamide, 1,4-dioxane, and combinations of any two or more thereof, more preferably toluene or tetrahydrofuran, and most preferably anhydrous tetrahydrofuran.

6. The method for preparing polyethylene glycol acetal derivative according to claim **1,** wherein the temperature for the activation treatment is preferably 20 to 60°C, more preferably 30 to 50°C, and most preferably 40°C; the duration of the activation treatment is preferably 6 to 24 hours, more preferably 8 to 12 hours; the molar ratio of small molecule acetal derivative **I-2** to the alkali reagent is preferably from 1:1 to 2:1; more preferably from 1:1 to 1.5 :1, and more preferably 1:1, 1.25:1, or 1.2:1.

7. The method for preparing polyethylene glycol acetal derivative according to claim **1,** wherein the molar ratio of polyethylene glycol derivative **I-1** containing a functional group X₁ to small molecule acetal derivative **I-2** is preferably from 1:10 to 1:160; when k is 1, the molar ratio of **I-1** to **I-2** is preferably from 1:10 to 1:30; the molar ratio of polyethylene glycol derivative **I-3** containing functional group X₁ to small molecule acetal derivative **I-2** is preferably from 1:20 to 1:100.

8. The method for preparing polyethylene glycol acetal derivative according to claim **1,** wherein the reaction is preferably conducted under an inert gas, and the inert gas is selected from the group consisting of nitrogen, helium, argon, and any combination thereof.

9. The method for preparing polyethylene glycol acetal derivative according to claim **1,** wherein the starting material **I-1** or **I-3** for the reaction is added dropwise to **I-2;** the purification process is conducted with precipitation or recrystallization; more preferably is conducted with recrystallization; the precipitating reagent is an ether reagent, selected from an ethyl ether or a methyl tert-butyl ether; the recrystallization reagent is selected from the group consisting of isopropanol, *n*-hexane, and any combination thereof; more preferably, the recrystallization reagent is a mixture of isopropanol and *n*-hexane, and the volume ratio of isopropanol to *n-*hexane is from 2:1 to 5:1.

10. A method for preparing polyethylene glycol aldehyde derivative, wherein the method comprises subjecting a polyethylene glycol acetal derivative, which is prepared by the methods of any one of claims **1-9,** to acid treatment.

11. The method for preparing polyethylene glycol aldehyde derivative according to claim **10,** wherein the acid is selected from the group consisting of hydrochloric acid, trifluoroacetic acid, formic acid, acetic acid, and mixtures thereof.

12. The method for preparing polyethylene glycol aldehyde derivative according to claim **10,** wherein the structures of the polyethylene glycolal derivatives comprises: 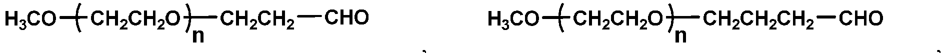 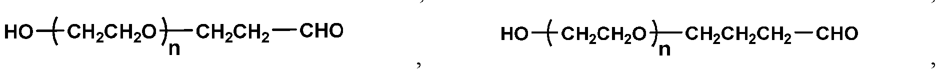 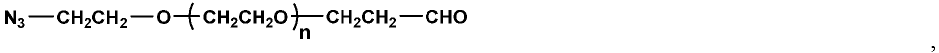 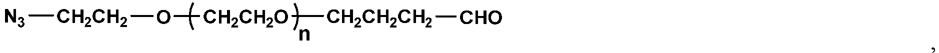 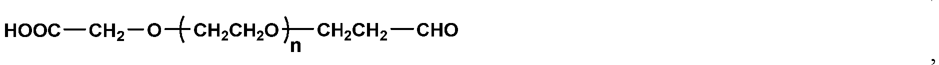 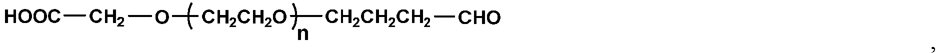 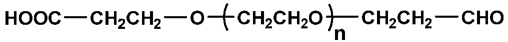 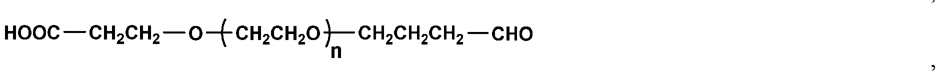 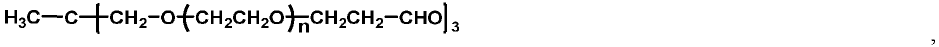 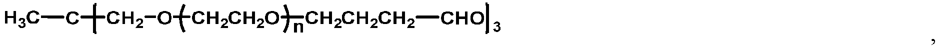 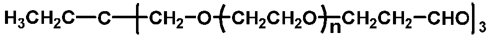 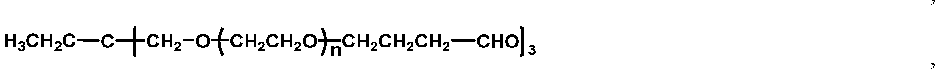 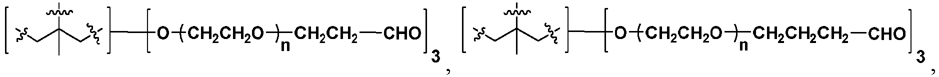 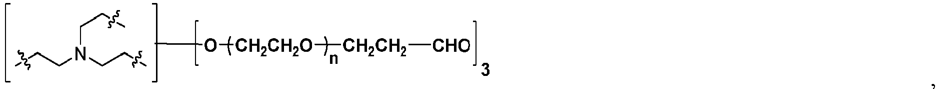 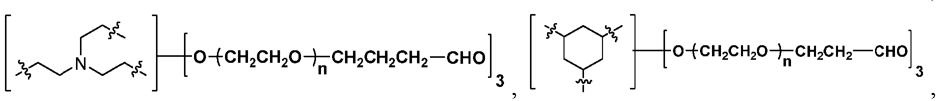 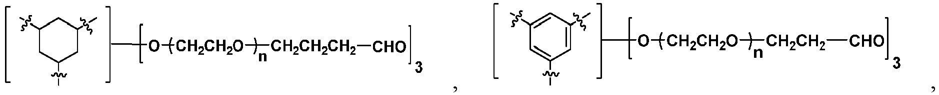 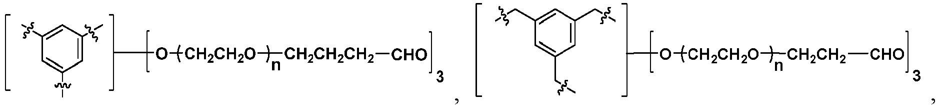 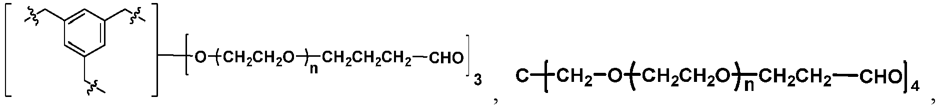 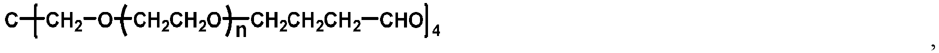 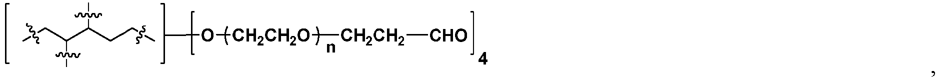 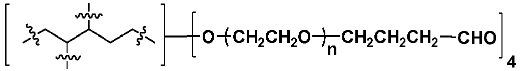 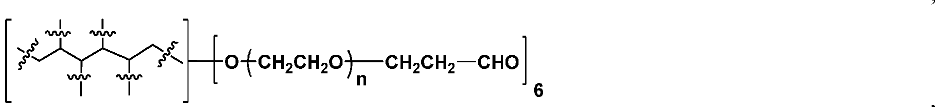 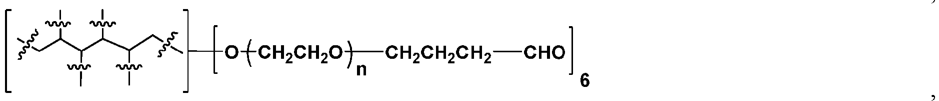 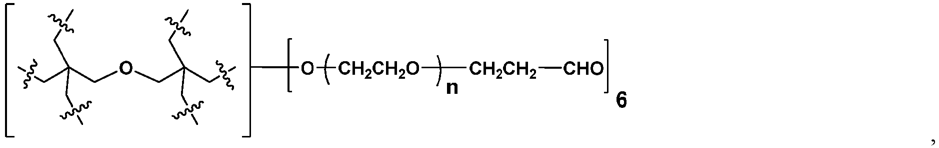 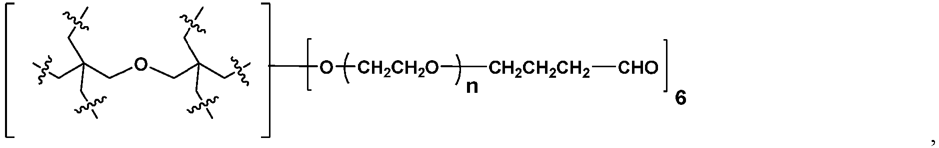 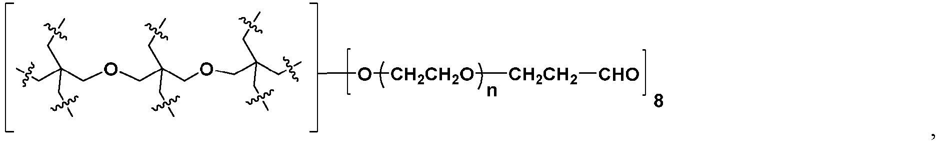 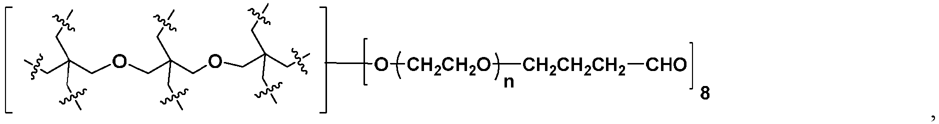 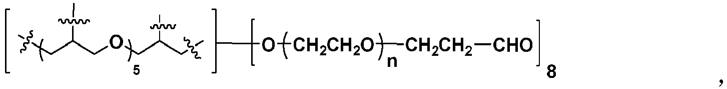 and

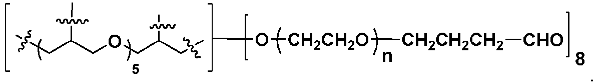

13. A bio-related substance modified with a polyethylene glycol aldehyde derivative prepared according to any one preparation method of claims **11-12,** wherein, the bio-related substance is selected from the group consisting of a peptide, a polypeptide, a protein, a polysaccharide, a steroid, a nucleotide, an oligonucleotide, a polynucleotide, and a lipid; more preferably, the bio-related substances contains an active amino group, or, after modification, contains at least one active amino group capable of coupling with the aldehyde group of the polyethylene glycol aldehyde derivative.

## Patentansprüche

1. Verfahren zur Herstellung eines Polyethylenglykolacetalderivats, wobei die Struktur des Derivats durch Formel (1) oder Formel (2) dargestellt ist: 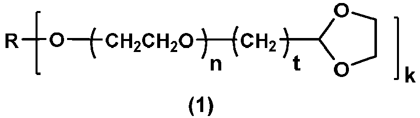 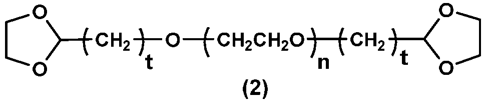
wobei n eine ganze Zahl von 20 bis 1000 ist;
t unabhängig eine ganze Zahl von 1 bis 6 bei jedem Auftreten ist;
k eine ganze Zahl von 1 bis 8 ist; wenn k 1 ist, R ausgewählt ist aus der Gruppe bestehend aus H, - CH₃, -CH₂CH₃, -TBS, -Bn, -(CH₂)ₜₚ-COOH, -(CH₂)ₜₚ-COOtBu, -(CH₂)ₜₚ-N₃; tp eine ganze Zahl von 1 bis 3 ist; wenn k eine ganze Zahl von 2 bis 8 ist, R eine mehrwertige verzweigte Struktur ist, die von zweiwertig bis achtwertig reicht;
das Herstellungsverfahren Folgendes umfasst:
Lösen von kleinmolekularem Acetalderivat **I-2** in einem organischen Lösungsmittel; Aktivieren von **I-2** mit einem Alkalireagens; Hinzufügen von Polyethylenglykolderivat **I-1** oder **I-3**, von denen jedes eine funktionelle Gruppe X₁ enthält, und Lösen in einem organischen Lösungsmittel; und dann Durchführen der Reaktion für 2 bis 24 Stunden bei 20 bis 90 °C, um das Polyethylenglykolacetalderivat zu erhalten, das durch Formel (1) oder Formel (2) dargestellt ist; wobei das Alkalireagens Natriumhydroxid oder Kaliumhydroxid ist;
wobei X₁ unabhängig eine p-Toluolsulfonylgruppe oder eine Methansulfonylgruppe bei jedem Auftreten ist; X₂ -OH ist; 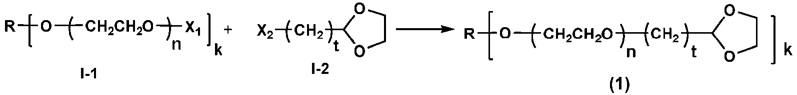 oder

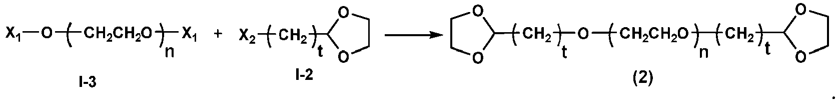

2. Verfahren zur Herstellung von Polyethylenglykolacetalderivat gemäß Anspruch **1,** wobei R ausgewählt ist aus der Gruppe bestehend aus H, -CH₃, -CH₂CH₃, -Bn, -(CH₂)ₜₚ-COOH, -(CH₂)ₜₚ-COOtBu, -(CH₂)ₜₚNH₂, -(CH₂)ₜₚNHCbz, -CH₂CH₂-,

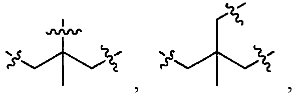

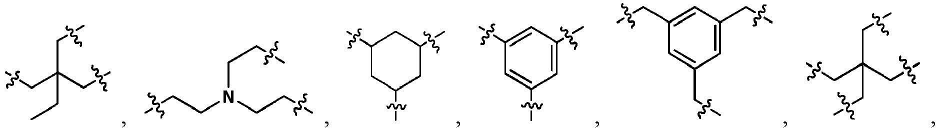

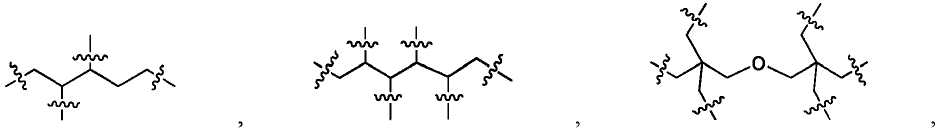

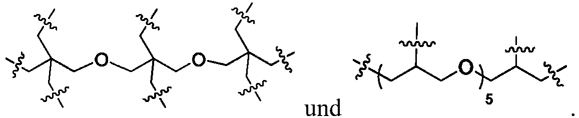

3. Verfahren zur Herstellung von Polyethylenglykolacetalderivat gemäß Anspruch 1, wobei das zahlenmittlere Molekulargewicht jeder Polyethylenglykolkette ausgewählt ist aus der Gruppe bestehend aus 900, 1000, 1500, 2000, 2500, 3000, 3350, 3400, 3500, 4000, 5000, 5500, 6000, 6500, 7000, 7500, 8000, 8500, 9000, 9500, 10000, 11000, 15000, 20000, 25000 und 30000, wobei das zahlenmittlere Molekulargewicht durch Gelpermeationschromatographie (GPC) bestimmt wird.

4. Verfahren zur Herstellung von Polyethylenglykolacetalderivat gemäß Anspruch 1, wobei, wenn X₁ eine Methansulfonylgruppe ist, X₂ -OH ist und das Herstellungsverfahren Folgendes umfasst: 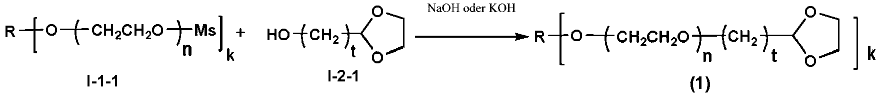 oder

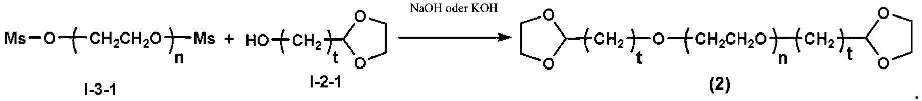

5. Verfahren zur Herstellung von Polyethylenglykolacetalderivat gemäß Anspruch **1,** wobei das organische Lösungsmittel ausgewählt ist aus der Gruppe bestehend aus Toluol, Dichlormethan, Chloroform, Acetonitril, Dimethylsulfoxid, Tetrahydrofuran, *N,N'-*Dimethylformamid, *N,N'*-Dimethylacetamid, 1,4-Dioxan und Kombinationen von beliebigen zwei oder mehr davon, bevorzugter Toluol oder Tetrahydrofuran und am meisten bevorzugt wasserfreiem Tetrahydrofuran.

6. Verfahren zur Herstellung von Polyethylenglykolacetalderivat gemäß Anspruch **1,** wobei die Temperatur für die Aktivierungsbehandlung bevorzugt 20 bis 60 °C, bevorzugter 30 bis 50 °C und am meisten bevorzugt 40 °C ist; die Dauer der Aktivierungsbehandlung bevorzugt 6 bis 24 Stunden, bevorzugter 8 bis 12 Stunden ist; das Molverhältnis von kleinmolekularem Acetalderivat **I-2** zu dem Alkalireagens bevorzugt 1:1 bis 2:1; bevorzugter 1:1 bis 1,5 :1 und bevorzugter 1:1, 1,25:1 oder 1,2:1 ist.

7. Verfahren zur Herstellung von Polyethylenglykolacetalderivat gemäß Anspruch 1, wobei das Molverhältnis von Polyethylenglykolderivat **I-1,** das eine funktionelle Gruppe X₁ enthält, zu kleinmolekularem Acetalderivat **I-2** bevorzugt 1:10 bis 1:160 ist; wenn k 1 ist, das Molverhältnis von **I-1** zu **I-2** bevorzugt 1:10 bis 1:30 ist; das Molverhältnis von Polyethylenglykolderivat **I-3**, das eine funktionelle Gruppe X₁ enthält, zu kleinmolekularem Acetalderivat **I-2** bevorzugt 1:20 bis 1:100 ist.

8. Verfahren zur Herstellung von Polyethylenglykolacetalderivat gemäß Anspruch **1,** wobei die Reaktion bevorzugt unter einem Inertgas durchgeführt wird und das Inertgas ausgewählt ist aus der Gruppe bestehend aus Stickstoff, Helium, Argon und einer beliebigen Kombination davon.

9. Verfahren zur Herstellung von Polyethylenglykolacetalderivat gemäß Anspruch **1,** wobei das Ausgangsmaterial **I-1** oder **I-3** für die Reaktion tropfenweise zu **I-2** hinzugefügt wird; der Reinigungsprozess mit Fällung oder Umkristallisation durchgeführt wird; bevorzugter mit Umkristallisation durchgeführt wird; das Fällungsreagens ein Etherreagens ist, ausgewählt aus einem Ethylether oder einem Methyl-tert-butylether; das Umkristallisationsreagens ausgewählt ist aus der Gruppe bestehend aus Isopropanol, *n*-Hexan und einer beliebigen Kombination davon; bevorzugter das Umkristallisationsreagens eine Mischung aus Isopropanol und *n*-Hexan ist und das Volumenverhältnis von Isopropanol zu *n*-Hexan 2:1 bis 5:1 ist.

10. Verfahren zur Herstellung von Polyethylenglykolaldehydderivat, wobei das Verfahren Unterziehen eines Polyethylenglykolacetalderivats, das durch die Verfahren nach einem der Ansprüche **1-9** hergestellt ist, einer Säurebehandlung umfasst.

11. Verfahren zur Herstellung von Polyethylenglykolaldehydderivat gemäß Anspruch **10,** wobei die Säure ausgewählt ist aus der Gruppe bestehend aus Salzsäure, Trifluoressigsäure, Ameisensäure, Essigsäure und Mischungen davon.

12. Verfahren zur Herstellung von Polyethylenglykolaldehydderivat gemäß Anspruch **10,** wobei die Strukturen der Polyethylenglykolderivate Folgendes umfassen: 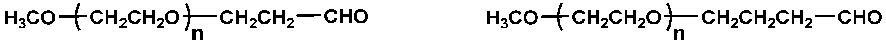 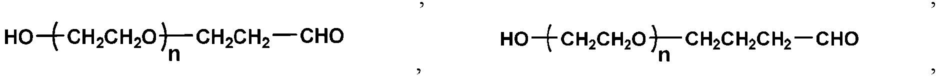 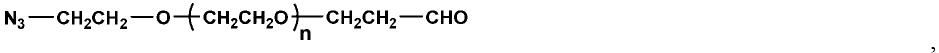 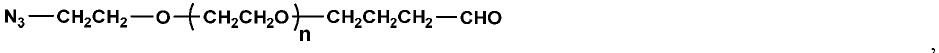 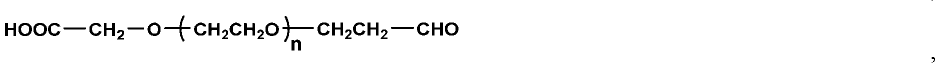 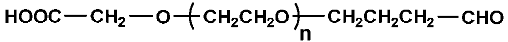 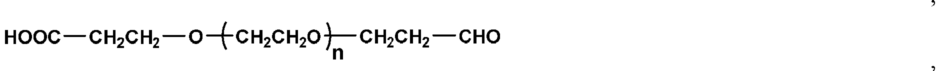 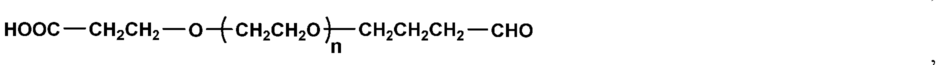 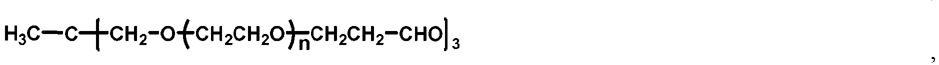 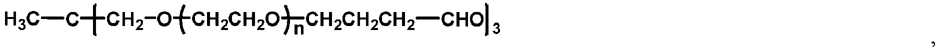 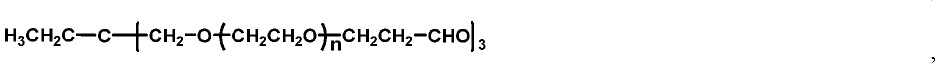 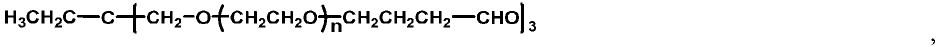 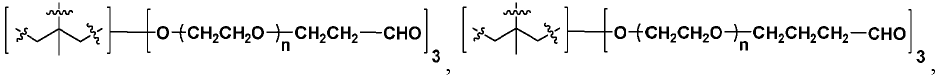 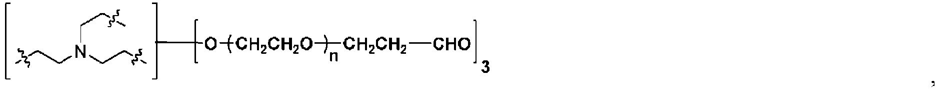 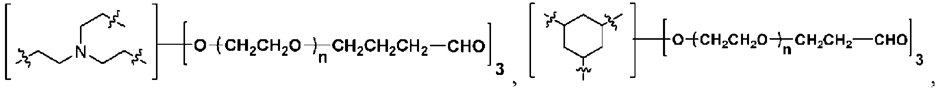 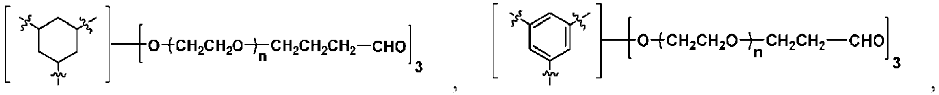 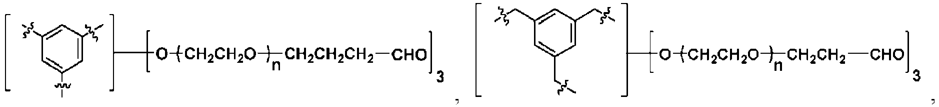 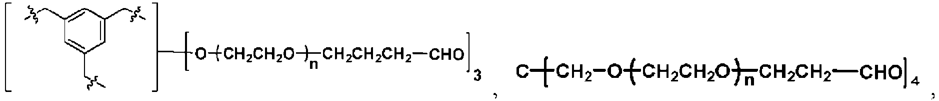 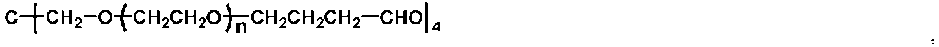 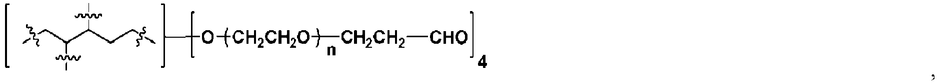 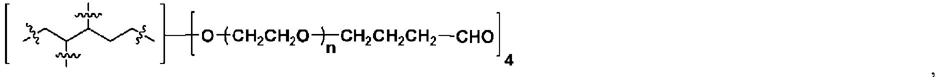 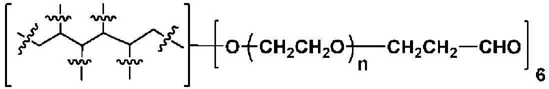 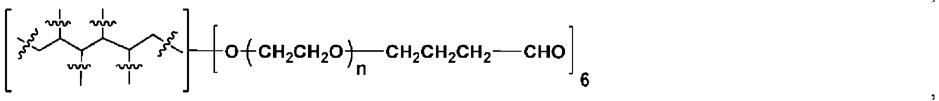 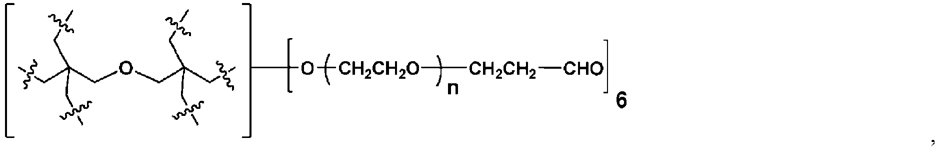 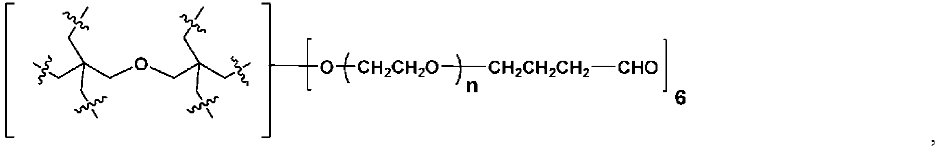 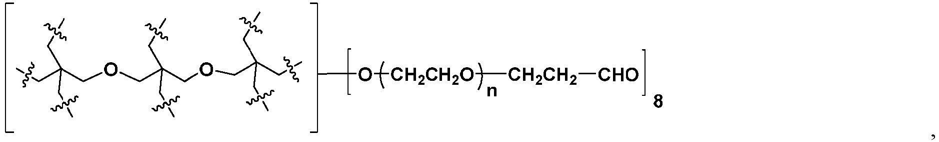 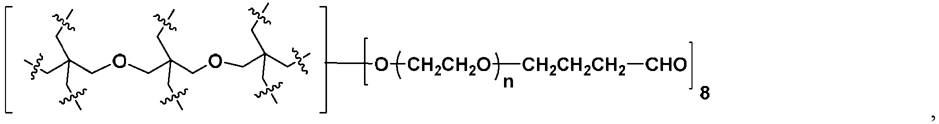 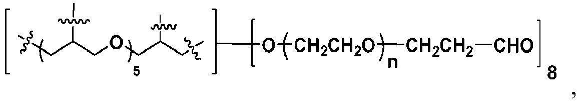 und

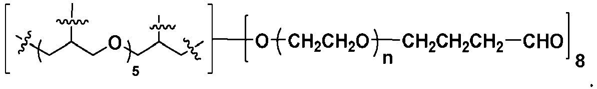

13. Bioverwandte Substanz, die mit einem Polyethylenglykolaldehydderivat modifiziert ist, das gemäß einem Herstellungsverfahren nach Anspruch **11-12** hergestellt ist, wobei die bioverwandte Substanz ausgewählt ist aus der Gruppe bestehend aus einem Peptid, einem Polypeptid, einem Protein, einem Polysaccharid, einem Steroid, einem Nukleotid, einem Oligonukleotid, einem Polynukleotid und einem Lipid; bevorzugter die bioverwandte Substanz eine aktive Aminogruppe enthält oder nach Modifizierung zumindest eine aktive Aminogruppe enthält, die in der Lage ist, mit der Aldehydgruppe des Polyethylenglykolaldehydderivats zu koppeln.

## Revendications

1. Procédé permettant la préparation d'un dérivé acétal de polyéthylène glycol, dans lequel la structure du dérivé est représentée par la formule (1) ou la formule (2) : 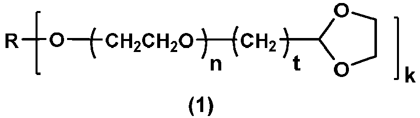 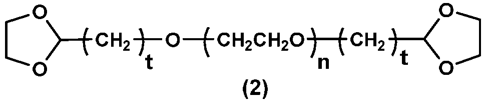
où, n est un entier de 20 à 1000 ;
t est indépendamment un entier de 1 à 6 à chaque occurrence ;
k est un entier de 1 à 8 ; lorsque k vaut 1, R est choisi dans le groupe constitué par H, -CH₃, -CH₂CH₃, -TBS, -Bn, -(CH₂)ₜₚ-COOH, -(CH₂)ₜₚ-COOtBu, -(CH₂)ₜₚ-N₃ ; tp est un entier de 1 à 3 ; lorsque k est un entier de 2 à 8, R est une structure ramifiée multivalente, allant de divalente à octavalente ;
le procédé de préparation comprend :
la dissolution de dérivé acétal de petite molécule **I-2** dans un solvant organique ; l'activation de **I-2** avec un réactif alcalin ; l'ajout de dérivé de polyéthylène glycol **I-1** ou **I-3,** dont chacun contient un groupe fonctionnel X₁ et la dissolution dans un solvant organique ; puis la réalisation de la réaction pendant 2 à 24 heures à 20 à 90 °C pour obtenir le dérivé acétal de polyéthylène glycol représenté par la formule (1) ou la formule (2) ; le réactif alcalin est l'hydroxyde de sodium ou l'hydroxyde de potassium ;
où, X₁ est indépendamment un groupe p-toluènesulfonyle ou un groupe méthanesulfonyle à chaque occurrence ; X₂ est -OH ; 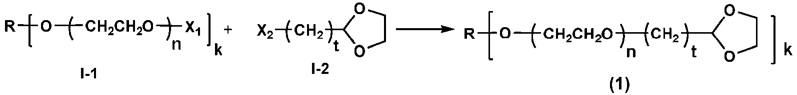 ou

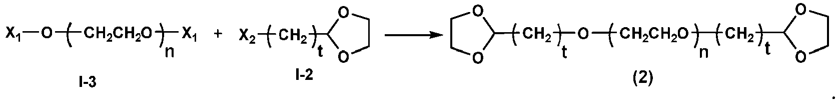

2. Procédé permettant la préparation de dérivé acétal de polyéthylène glycol selon la revendication **1,** dans lequel R est choisi dans le groupe constitué par H, -CH₃, -CH₂CH₃, -Bn, - (CH₂)ₜₚ-COOH, -(CH₂)ₜₚ-COOtBu, -(CH₂)ₜₚNH₂, -(CH₂)ₜₚNHCbz, -CH₂CH₂-,

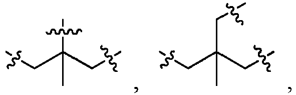

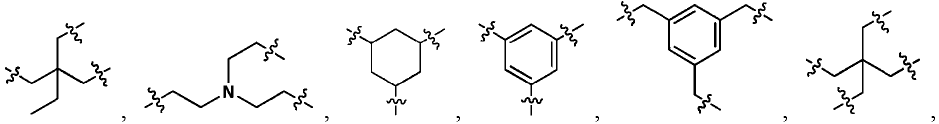

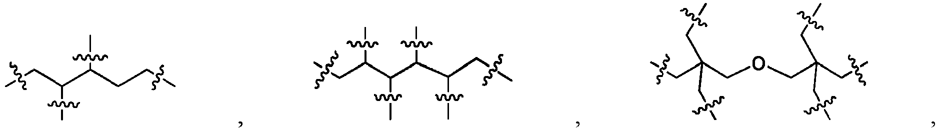

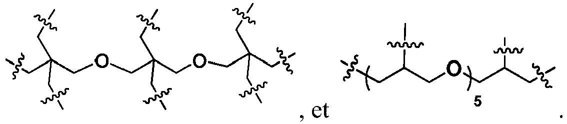

3. Procédé permettant la préparation de dérivé acétal de polyéthylène glycol selon la revendication 1, dans lequel le poids moléculaire moyen en nombre de chaque chaîne de polyéthylène glycol est choisi dans le groupe constitué de 900, 1 000, 1 500, 2 000, 2 500, 3 000, 3 350, 3 400, 3 500, 4 000, 5 000, 5 500, 6 000, 6 500, 7 000, 7 500, 8 000, 8 500, 9 000, 9 500, 10 000, 11 000, 15 000, 20 000, 25 000 et 30 000, dans lequel le poids moléculaire moyen en nombre est déterminé par chromatographie par perméation sur gel (GPC).

4. Procédé permettant la préparation de dérivé acétal de polyéthylène glycol selon la revendication **1,** dans lequel lorsque X₁ est un groupe méthanesulfonyle, X₂ est -OH, et le procédé de préparation comprend : 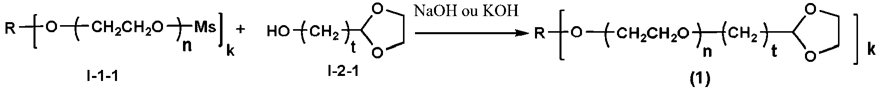 ou

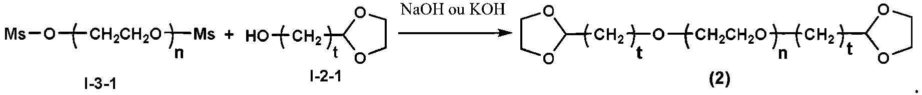

5. Procédé permettant la préparation de dérivé acétal de polyéthylène glycol selon la revendication **1,** dans lequel le solvant organique est choisi dans le groupe constitué par le toluène, le dichlorométhane, le chloroforme, l'acétonitrile, le diméthylsulfoxyde, le tétrahydrofurane, le *N,N'*-diméthylformamide, le *N,N'*-diméthylacétamide, le 1,4-dioxane, et des combinaisons de deux quelconques de ceux-ci ou plus, idéalement le toluène ou le tétrahydrofurane, et plus préférablement le tétrahydrofurane anhydre.

6. Procédé permettant la préparation de dérivé acétal de polyéthylène glycol selon la revendication **1,** dans lequel la température pour le traitement d'activation est de préférence de 20 à 60 °C, idéalement de 30 à 50 °C, et plus préférablement de 40 °C ; la durée du traitement d'activation est de préférence de 6 à 24 heures, idéalement de 8 à 12 heures ; le rapport molaire du dérivé acétal de petite molécule **I-2** au réactif alcalin est de préférence de 1:1 à 2:1 ; idéalement de 1:1 à 1,5:1, et idéalement de 1:1, 1,25:1, ou 1,2:1.

7. Procédé permettant la préparation de dérivé acétal de polyéthylène glycol selon la revendication **1,** dans lequel le rapport molaire du dérivé de polyéthylène glycol **I-1** contenant un groupe fonctionnel X₁ au dérivé acétal de petite molécule **I-2** est de préférence de 1:10 à 1:160 ; lorsque k est 1, le rapport molaire de **I-1** à **I-2** est de préférence de 1:10 à 1:30 ; le rapport molaire du dérivé de polyéthylène glycol **I-3** contenant un groupe fonctionnel X₁ au dérivé acétal de petite molécule **I-2** est de préférence de 1:20 à 1:100.

8. Procédé pour la préparation de dérivé acétal de polyéthylène glycol selon la revendication **1,** dans lequel la réaction est de préférence réalisée sous un gaz inerte, et le gaz inerte est choisi dans le groupe constitué par l'azote, l'hélium, l'argon et toute combinaison de ceux-ci.

9. Procédé permettant la préparation de dérivé acétal de polyéthylène glycol selon la revendication **1,** dans lequel le matériau de départ **I-1** ou **I-3** pour la réaction est ajouté goutte à goutte à **I-2 ;** le processus de purification est réalisé par précipitation ou recristallisation ; idéalement, il est réalisé par recristallisation ; le réactif de précipitation est un réactif éther, choisi parmi un éther éthylique ou un éther méthyl-tert-butylique ; le réactif de recristallisation est choisi dans le groupe constitué par l'isopropanol, le *n*-hexane, et toute combinaison de ceux-ci ; idéalement, le réactif de recristallisation est un mélange d'isopropanol et de *n*-hexane, et le rapport volumique d'isopropanol au *n*-hexane est de 2:1 à 5:1.

10. Procédé permettant la préparation de dérivé aldéhyde de polyéthylène glycol, dans lequel le procédé comprend la soumission d'un dérivé acétal de polyéthylène glycol, qui est préparé par les procédés de l'une quelconque des revendications **1** à **9,** à un traitement acide.

11. Procédé permettant la préparation de dérivé aldéhyde de polyéthylène glycol selon la revendication **10,** dans lequel l'acide est choisi dans le groupe constitué de l'acide chlorhydrique, l'acide trifluoroacétique, l'acide formique, l'acide acétique et de mélanges de ceux-ci.

12. Procédé permettant la préparation de dérivé aldéhyde de polyéthylène glycol selon la revendication **10,** dans lequel les structures des dérivés de polyéthylène glycol comprennent : 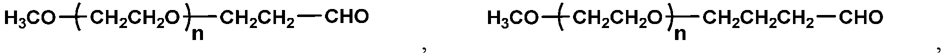 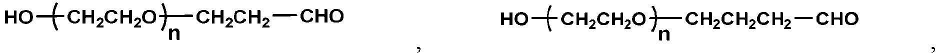 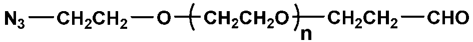 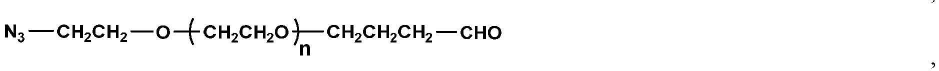 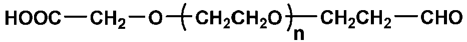 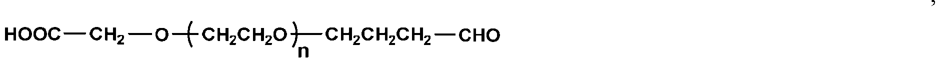 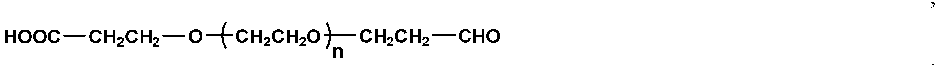 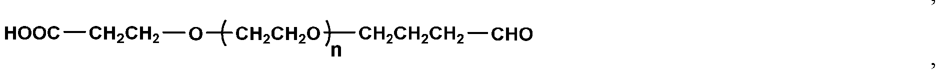 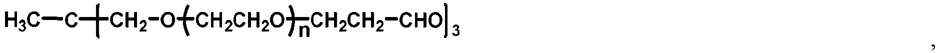 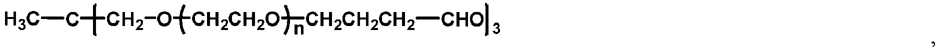 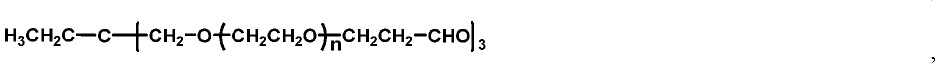 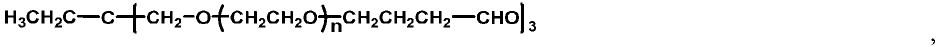 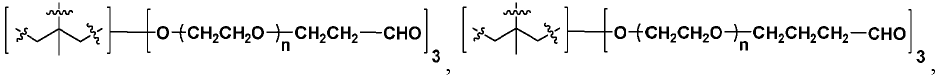 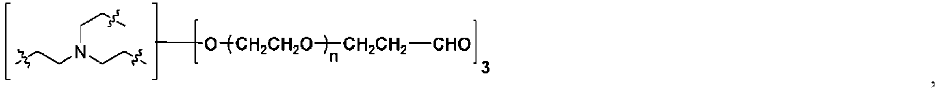 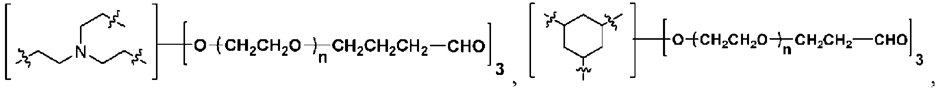 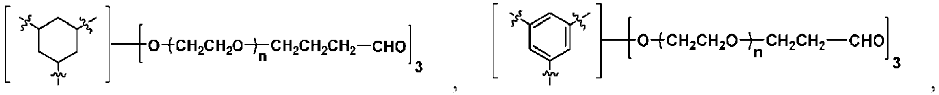 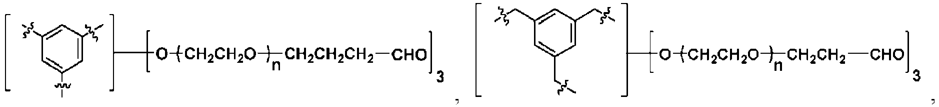 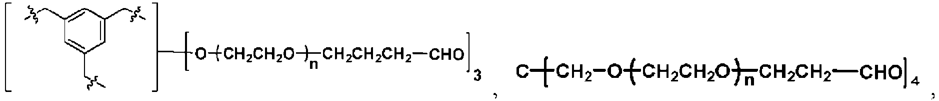 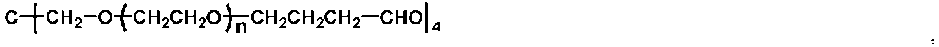 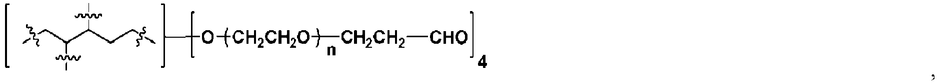 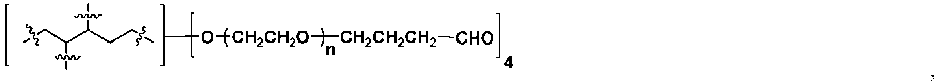 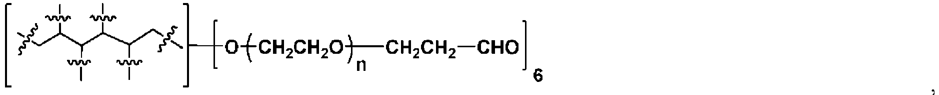 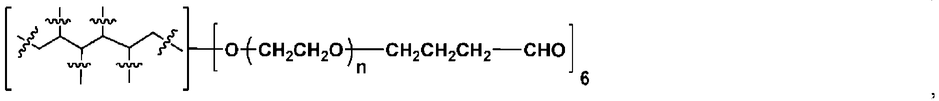 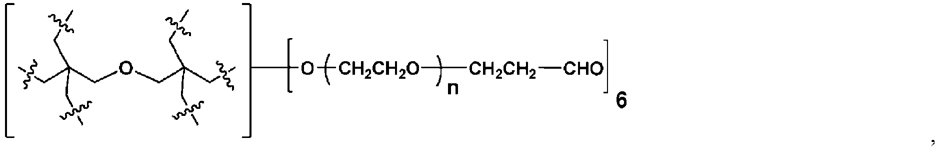 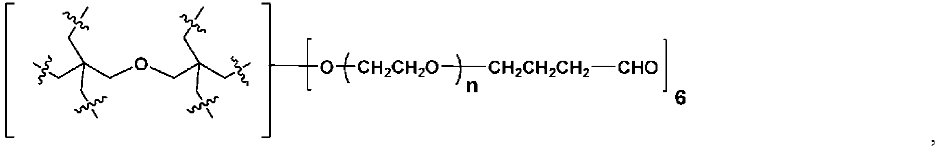 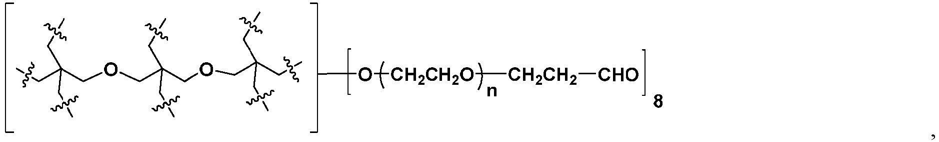 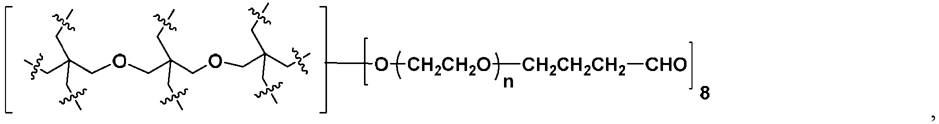 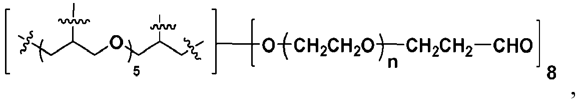 et

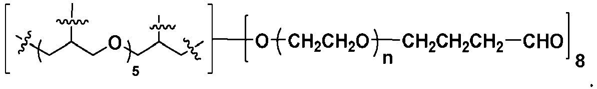

13. Substance d'origine biologique modifiée avec un dérivé aldéhyde de polyéthylène glycol préparé selon l'un quelconque des procédés de préparation des revendications **11 à 12,** ladite substance d'origine biologique étant choisie dans le groupe constitué d'un peptide, d'un polypeptide, d'une protéine, d'un polysaccharide, d'un stéroïde, d'un nucléotide, d'un oligonucléotide, d'un polynucléotide et d'un lipide ; idéalement, les substances d'origine biologique contiennent un groupe amino actif, ou, après modification, contiennent au moins un groupe amino actif capable de se coupler avec le groupe aldéhyde du dérivé aldéhyde de polyéthylène glycol.
